# EUROPEAN PATENT APPLICATION

(11) **EP 3 709 375 A1**
(43) Date of publication of application: **16.09.2020**
(21) Application number: 18872125.2
(22) Date of filing: 05.11.2018
(51) Int. Cl.: H01L 51/50, C07F 15/00, C09K 11/06, C07D 209/94

(54) **COMPOUND HAVING INDENOCARBAZOLE RING STRUCTURE, AND ORGANIC ELECTROLUMINESCENT ELEMENT**

(30) Priority: 06.11.2017 JP 2017213378
(71) Applicant: Hodogaya Chemical Co., Ltd., Chuo-ku, Tokyo 104-0028 (JP)
(72) Inventor: MOCHIZUKI, Shunji, Tokyo 104-0028 (JP); KASE, Kouki, Tokyo 1040028 (JP); YAMAMOTO, Takeshi, Tokyo 104-0028 (JP); HAYASHI, Shuichi, Tokyo 104-0028 (JP)
(74) Representative: Müller-Boré & Partner Patentanwälte PartG mbB
(86) International application number: PCT/JP2018/040962
(87) International publication number: WO 2019/088281

(57) **Abstract**

[Object] It is an object of the present invention to provide an organic compound having excellent properties such as excellent hole injection/transport performance, electron blocking performance, high stability in a thin-film state, and high light emission efficiency as a material for a highly efficient organic EL device having a high durability, and a highly efficient organic EL device having a high durability by using this compound.

[Solving Means] An organic EL device, including, between an anode and a cathode, at least a first hole transport layer, a second hole transport layer, a green light-emitting layer, and an electron transport layer in the stated order from a side of the anode, the organic EL device being characterized in that the second hole transport layer, or at least one of stacked films disposed between the first hole transport layer and the electron transport layer contains a compound having an indenocarbazole ring structure, the compound being represented by the following general formula (1). 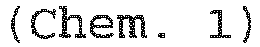 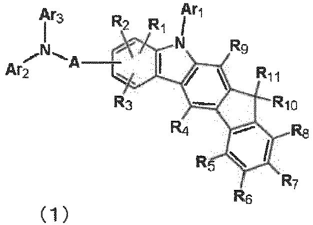 (In the formula, A represents a divalent group of a substituted or unsubstituted aromatic hydrocarbon, a divalent group of a substituted or unsubstituted aromatic heterocycle, or a divalent group of a substituted or unsubstituted fused polycyclic aromatic. Ar₁, Ar₂, and Ar₃ may be the same as or different from each other, and each represent a substituted or unsubstituted aromatic hydrocarbon group, a substituted or unsubstituted aromatic heterocyclic group, or a substituted or unsubstituted fused polycyclic aromatic group. Here, A and Ar₂ or Ar₂ and Ar₃ may form a ring with a single bond or may be bonded to each other via a substituted or unsubstituted methylene group, an oxygen atom, or a sulfur atom to form a ring. R₁ to R₉ may be the same as or different from each other, each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring. R₁₀ and R₁₁ may be the same as or different from each other, each represent a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group., a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring.)

## Description

### Technical Field

The present invention relates to a compound suitable for an organic electroluminescence device (hereinafter, abbreviated as an organic EL device) that is a self-light-emitting device suitable for various display devices, and to the device, and specifically to a compound having an indenocarbazole ring structure and an organic EL device that uses the compound.

### Background Art

Since the organic EL device is a self-light-emitting device, it is brighter than the liquid crystal device and excellent in visibility, and capable of performing clear display, and thus, active research has been done thereon.

In 1987, C.W.Tang et al. (Eastman Kodak Company) have developed a stacked structural device in which various roles are assigned to the materials, and put an organic EL device using an organic material to practical use. They have stacked a fluorophore capable of transporting electrons and an aromatic amine compound capable of transporting tris(8-hydroxyquinoline) aluminum (hereinafter, abbreviated as Alq₃) and holes, and injected both charges into a fluorophore layer to emit light, thereby achieving high luminance of 1000 cd/m² or more with a voltage of 10 V or less (see, for example, Patent Literature 1 and Patent Literature 2).

Many improvements have been made for practical use of the organic EL device until now. In an electroluminescence device that subdivides the various roles and includes an anode, a hole injection layer, a hole transport layer, a light-emitting layer, an electron transport layer, an electron injection layer, and a cathode in the stated order on a substrate, high efficiency and durability have been achieved (see, for example, Non-Patent Literature 1).

Further, for the purpose of further improving the light emission efficiency, attempts have been made to use a triplet exciton and utilization of a phosphorescent emitter is being considered (see, for example, Non-Patent Literature 2).

The light-emitting layer can also be prepared by doping a charge transport compound generally called a host material with a fluorophore or a phosphorescent emitter. In recent years, a highly efficient organic EL device that uses an iridium complex as a phosphorescent material and a compound having a carbazole structure as a host material has been proposed (see, for example, Patent Literature 3).

Further, both a compound having a nitrogen-containing heteroaromatic ring structure with high electron transportability and a compound having a carbazole structure having hole transport ability are used as hosts to increase the transportability of electron and holes and improve the light emission efficiency has been remarkably as compared with the case of using one of them alone (see, for example, Patent Literature 5).

As a hole transport material and a host material having hole transportability that have been used for a phosphorescent organic EL device, biscarbazole derivatives (e.g., HTM-1) and triscarbazole derivatives (e.g., HTM-2), which are disclosed in Patent Literature 6 and Patent Literature 7, have been proposed.

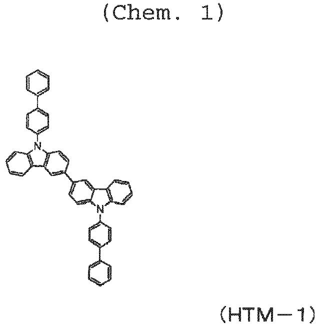

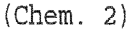

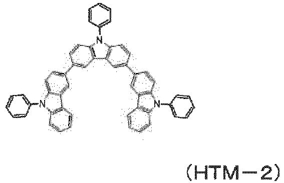

These compounds have an excellent electron blocking property, but have a problem of low mobility of holes. For this reason, in the case where a light-emitting layer having improved electron transportability is combined with them, there is a concern that the supply of holes to the light-emitting layer is rate-limiting and the number of electrons in the light-emitting layer is biased toward an excess.

In a device that uses these compounds for a hole transport layer and a light-emitting layer, the number of electrons in the light-emitting layer is biased toward an excess, and the current efficiency and the device lifetime are not sufficient. For this reason, a hole transport material and a host material having a high mobility of holes and an excellent durability to electrons have been desired.

### Citation List

### Patent Literature

Patent Literature 1: Japanese Patent Application Laid-open No. 1996-048656
Patent Literature 2: Japanese Patent Application Laid-open No. 1995-126615
Patent Literature 3: Japanese Patent Application Laid-open No. 2006-151979
Patent Literature 4: WO 2015/034125
Patent Literature 5: WO 2016/013732
Patent Literature 6: Japanese Patent Application Laid-open No. 1996-003547
Patent Literature 7: WO 2012/001986
Patent Literature 8: WO 2012/014500
Patent Literature 9: Japanese Patent Application Laid-open No. 2002-105055
Patent Literature 10: WO 2014/007565
Patent Literature 11: WO 2014/188947
Patent Literature 12: WO 2015/190400
Patent Literature 13: Japanese Patent Application Laid-open No. 2010-83862
Patent Literature 14: WO 2015/038503
Patent Literature 15: Japanese Patent Application Laid-open No. 2005-108804
Patent Literature 16: WO 2014/009310

### Non-Patent Literature

Non-Patent Literature 1: The Japan Society of Applied Physics, proceedings of the ninth workshop, pp. 55-61 (2001)
Non-Patent Literature 2: The Japan Society of Applied Physics, proceedings of the ninth workshop, pp. 23-31 (2001)
Non-Patent Literature 3: J.Org.chcm.,71,1802(2006)
Non-Patent Literature 4: J.Org.chcm.,79,6310(2014)

### Disclosure of Invention

### Technical Problem

It is an object of the present invention to provide an organic compound having excellent properties such as excellent hole injection/transport performance, electron blocking performance, high stability in a thin-film state, and high light emission efficiency as a material for a highly efficient organic EL device having a high durability, and a highly efficient organic EL device having a high durability by using this compound.

Examples of the physical properties that an organic compound to be provided by the present invention should have include (1) having a high hole injection property, (2) having a high mobility of holes, (3) having excellent electron blocking performance, (4) having high stability in a thin-film state, and (5) having an excellent durability to electrons. Further, examples of the physical properties that an organic EL device to be provided by the present invention should have include (1) having high light emission efficiency and (2) having a long device lifetime.

### Solution to Problem

In view of the above, in order to achieve the above-mentioned object, the present inventors have focused on that a high mobility of holes can be expected by the planarity of an indenocarbazole ring structure, a high triplet energy level can be expected, excellent electron blocking property can be expected, an excellent durability to electrons and excellent stability in a thin-film state can be expected, and an aromatic tertiary amine structure has high hole injection/transport performance, and have designed and chemically synthesized a compound having an indenocarbazole ring structure and a compound having an aromatic tertiary amine structure. Various organic EL devices have been prototyped using the compound, and the properties of the device were intensively evaluated. As a result, the present invention was completed.
[1] An organic EL device, including, between an anode and a cathode, at least a first hole transport layer, a second hole transport layer, a green light-emitting layer, and an electron transport layer in the stated order from a side of the anode, the organic EL device being characterized in that the second hole transport layer, or at least one of stacked films disposed between the first hole transport layer and the electron transport layer contains a compound having an indenocarbazole ring structure, the compound being represented by the following general formula (1). 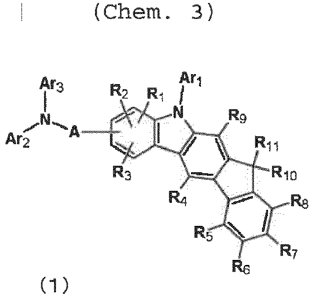 (In the formula, A represents a divalent group of a substituted or unsubstituted aromatic hydrocarbon, a divalent group of a substituted or unsubstituted aromatic heterocycle, or a divalent group of a substituted or unsubstituted fused polycyclic aromatic. Ar₁, Ar₂, and Ar₃ may be the same as or different from each other, and each represent a substituted or unsubstituted aromatic hydrocarbon group, a substituted or unsubstituted aromatic heterocyclic group, or a substituted or unsubstituted fused polycyclic aromatic group. Here, A and Ar₂ or Ar₂ and Ar₃ may form a ring with a single bond or may be bonded to each other via a substituted or unsubstituted methylene group, an oxygen atom, or a sulfur atom to form a ring. R₁ to R₉ may be the same as or different from each other, each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring. R₁₀ and R₁₁ may be the same as or different from each other, each represent a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring.)
[2] An organic EL device including, between an anode and a cathode, at least a first hole transport layer, a second hole transport layer, a green light-emitting layer, and an electron transport layer in the stated order from a side of the anode, the organic EL device being characterized in that the second hole transport layer, or at least one of stacked films disposed between the first hole transport layer and the electron transport layer contains a compound having an indenocarbazole ring structure, the compound being represented by the following general formula (2). 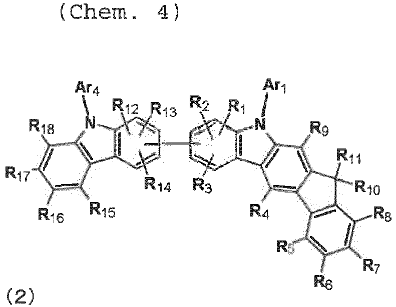 (In the formula, Ar₁ and Ar₄ may be the same as or different from each other, and each represent a substituted or unsubstituted aromatic hydrocarbon group, a substituted or unsubstituted aromatic heterocyclic group, or a substituted or unsubstituted fused polycyclic aromatic group. R₁ to R₉ and R₁₂ to R₁₈ may be the same as or different from each other, each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring. R₁₀ and R₁₁ may be the same as or different from each other, each represent a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring.)
[3] The organic EL device according to [1] or [2] above, characterized in that
   the green light-emitting layer contains a host and a phosphorescent dopant, and the host contains at least one first host compound represented by the following chemical formula Host-A and at least one second host compound represented by the following chemical formula Host-B. 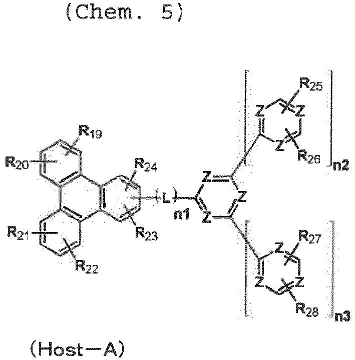 (In the Host-A, Zs each independently represent N or CRa, and at least one of Zs represents N. R₁₉ to R₂₈ and Ra each independently represent a hydrogen atom, a deuterium atom, a fluorine atom, a chlorine atom, a cyano group, a nitro group, a substituted or unsubstituted alkyl group having 1 to 15 carbon atoms, or a substituted or unsubstituted aryl group having 6 to 12 ring carbon atoms. The total number of 6-membered rings substituted with triphenylene groups in the Host-A is six or less. L represents a substituted or unsubstituted phenylene group, a substituted or unsubstituted biphenylene group, or a substituted or unsubstituted terphenylene group. n1 to n3 each independently represent 0 or 1, and n1+n2+n3≥1.) 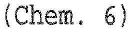 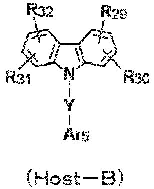 (In the Host-B, Y represents a single bond, a substituted or unsubstituted arylene group having 6 to 30 ring carbon atoms, or a substituted or unsubstituted heteroarylene group having 5 to 30 ring carbon atoms. Ar₅ represents a substituted or unsubstituted aryl group having 6 to 30 ring carbon atoms or a substituted or unsubstituted heteroaryl group having 5 to 30 ring carbon atoms. R₂₉ to R₃₂ each independently represent a hydrogen atom, a deuterium atom, a fluorine atom, a chlorine atom, a cyano group, a nitro group, an alkyl group having 1 to 15 carbon atoms, a substituted or unsubstituted aryl group having 6 to 50 ring carbon atoms, or a substituted or unsubstituted heteroaryl group having 4 to 50 ring carbon atoms. At least one of R₂₉ to R₃₂ and Ar₅ includes a substituted or unsubstituted triphenylene group or a substituted or unsubstituted carbazole group.)
[4] The organic EL device according to any one of [1] to [3] above, characterized in that the green light-emitting layer contains a host and a phosphorescent dopant, and the phosphorescent dopant is a metal complex containing iridium.
[5] The organic EL device according to any one of [1] to [3] above, characterized in that the green light-emitting layer contains a host and a phosphorescent dopant, and the phosphorescent dopant is a metal complex represented by the following general formula (3). 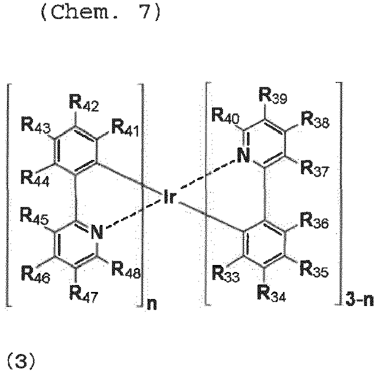 (In the formula, R₃₃ to R₄₈ may be the same as or different from each other, and each represent a hydrogen atom, a deuterium atom, a fluorine atom, a chlorine atom, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a trimethylsilyl group, a substituted or unsubstituted aromatic hydrocarbon group, a substituted or unsubstituted aromatic heterocyclic group, a substituted or unsubstituted fused polycyclic aromatic group, a substituted or unsubstituted aryloxy group, or a disubstituted amino group substituted with a group selected from an aromatic hydrocarbon group, an aromatic heterocyclic group, or a fused polycyclic aromatic group. n represents an integer of 1 to 3.)
[6] The organic EL device according to any one of [1] to [5] above, characterized in that
   the electron transport layer contains a compound having a pyrimidine structure, the compound being represented by the following general formula (4). 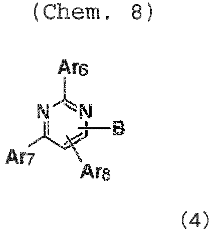 (In the formula, Ar₆ represents a substituted or unsubstituted aromatic hydrocarbon group or a substituted or unsubstituted fused polycyclic aromatic group. Ar₇ and Ar₈ may be the same as or different from each other, and each represent a hydrogen atom, a substituted or unsubstituted aromatic hydrocarbon group, or a substituted or unsubstituted fused polycyclic aromatic group. B represents a monovalent group represented by the following structural formula (5). Here, there is no case that both Ar₇ and Ar₈ are hydrogen atoms.) 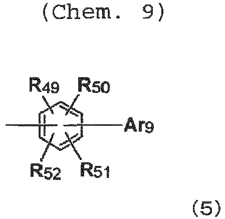 (In the formula, Ar₉ represents a substituted or unsubstituted aromatic heterocyclic group. R₄₉ to R₅₂ may be the same as or different from each other, and each represent a hydrogen atom, a deuterium atom, a fluorine atom, a chlorine atom, a cyano group, a trifluoromethyl group, a linear or branched alkyl group having 1 to 6 carbon atoms, a substituted or unsubstituted aromatic hydrocarbon group, a substituted or unsubstituted aromatic heterocyclic group, or a substituted or unsubstituted fused polycyclic aromatic group.)
[7] The organic EL device according to any one of [1] to [5] above, characterized in that
   the electron transport layer contains a compound having a benzoazole structure, the compound being represented by the following general formula (6). 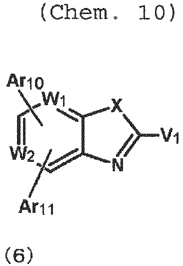 (In the formula, AR₁₀ and AR₁₁ may be the same as or different from each other, and each represent a hydrogen atom, a deuterium atom, a substituted or unsubstituted aromatic hydrocarbon group, a substituted or unsubstituted fused polycyclic aromatic group, or a substituted or unsubstituted aromatic heterocyclic group. V₁ represents a substituted or unsubstituted aromatic hydrocarbon group, a substituted or unsubstituted fused polycyclic aromatic group, a substituted or unsubstituted aromatic heterocyclic group, a linear or branched alkyl group having 1 to 6 carbon atoms which may have a substituted group, a cycloalkyl group having 5 to 10 carbon atoms which may have a substituted group, or a linear or branched alkenyl group having 2 to 6 carbon atoms which may have a substituted group. X represents an oxygen atom or a sulfur atom. W₁ and W₂ may be the same as or different from each other, and each represent a carbon atom or a nitrogen atom.)
[8] The organic EL device according to any one of [1] to [7] above, characterized in that the first hole transport layer contains a triphenylamine derivative represented by the following general formula (7) or the following general formula (8). 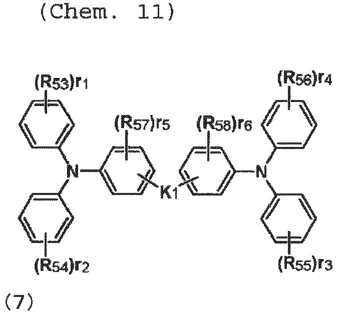
(In the formula, R₅₃ to R₅₈ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₁ to r₆ may be the same as or different from each other, r₁ to r₄ each represent an integer of 0 to 5, and r₅ and r₆ each represent an integer of 0 to 4. In a case where any of r₁ to r₆ is an integer of two or more, a plurality of R₅₃, a plurality of R₅₄, a plurality of R₅₅, a plurality of R₅₆, a plurality of R₅₇, or a plurality of R₅₈ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. K₁ represents a divalent group represented by any of the following structural formulae (HTM-A) to (HTM-F) or a single bond.) 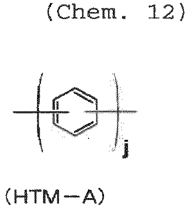 (In the formula, j represents an integer of 1 to 3) 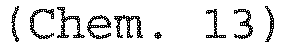 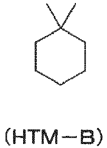 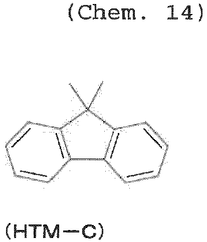 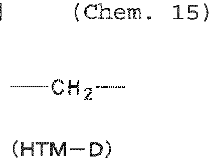 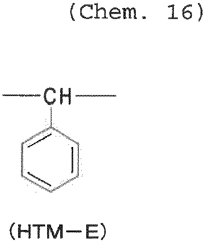 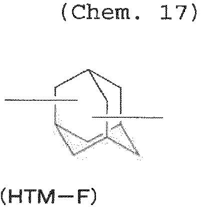 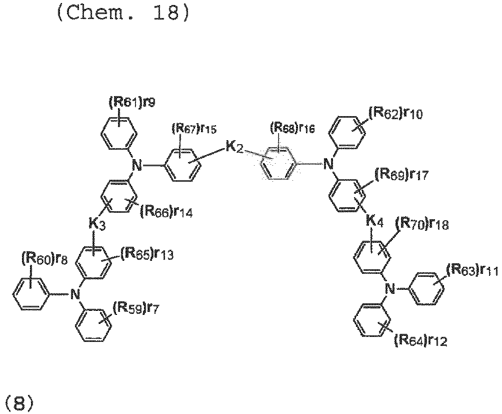 (In the formula, R₅₉ to R₇₀ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₇ to r₁₈ may be the same as or different from each other, r₇ to r₁₂ each represent an integer of 0 to 5, and r₁₃ to r₁₈ each represent an integer of 0 to 4. In a case where any of r₇ to r₁₈ is an integer of two or more, a plurality of R₅₉, a plurality of R₆₀, a plurality of R₆₁, a plurality of R₆₂, a plurality of R₆₃, a plurality of R₆₄, a plurality of R₆₅, a plurality of R₆₆, a plurality of R₆₇, a plurality of R₆₈, a plurality of R₆₉, or a plurality of R₇₀ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. K₂ to K₄ may be the same as or different from each other, and each represent a divalent group represented by any of the structural formulae (HTM-A) to (HTM-F) in the general formula (7), or a single bond.)

### Brief Description of Drawings

[Fig. 1] Fig. 1 is a diagram showing Compound (1-1) to Compound (1-12) as favorable specific examples of a compound having an indenocarbazole ring structure, the compound being represented by the general formula (1).
[Fig. 2] Fig. 2 is a diagram showing Compound (1-13) to Compound (1-24) as favorable specific examples of a compound having an indenocarbazole ring structure, the compound being represented by the general formula (1) .
[Fig. 3] Fig. 3 is a diagram showing Compound (1-25) to Compound (1-36) as favorable specific examples of a compound having an indenocarbazole ring structure, the compound being represented by the general formula (1) .
[Fig. 4] Fig. 4 is a diagram showing Compound (1-37) to Compound (1-48) as favorable specific examples of a compound having an indenocarbazole ring structure, the compound being represented by the general formula (1) .
[Fig. 5] Fig. 5 is a diagram showing Compound (1-49) to Compound (1-57) as favorable specific examples of a compound having an indenocarbazole ring structure, the compound being represented by the general formula (1) .
[Fig. 6] Fig. 6 is a diagram showing Compound (A-1) to Compound (A-12) as favorable specific examples of a compound represented by the chemical formula (Host-A).
[Fig. 7] Fig. 7 is a diagram showing Compound (A-13) to Compound (A-24) as favorable specific examples of a compound represented by the chemical formula (Host-A).
[Fig. 8] Fig. 8 is a diagram showing Compound (A-25) to Compound (A-36) as favorable specific examples of a compound represented by the chemical formula (Host-A).
[Fig. 9] Fig. 9 is a diagram showing Compound (A-37) to Compound (A-48) as favorable specific examples of a compound represented by the chemical formula (Host-A).
[Fig. 10] Fig. 10 is a diagram showing Compound (A-49) to Compound (A-57) as favorable specific examples of a compound represented by the chemical formula (Host-A).
[Fig. 11] Fig. 11 is a diagram showing Compound (B-1) to Compound (B-12) as favorable specific examples of a compound represented by the chemical formula (Host-B).
[Fig. 12] Fig. 12 is a diagram showing Compound (B-13) to Compound (B-24) as favorable specific examples of a compound represented by the chemical formula (Host-B).
[Fig. 13] Fig. 13 is a diagram showing Compound (B-25) to Compound (B-36) as favorable specific examples of a compound represented by the chemical formula (Host-B).
[Fig. 14] Fig. 14 is a diagram showing Compound (B-37) to Compound (B-48) as favorable specific examples of a compound represented by the chemical formula (Host-B).
[Fig. 15] Fig. 15 is a diagram showing Compound (B-49) to Compound (B-60) as favorable specific examples of a compound represented by the chemical formula (Host-B).
[Fig. 16] Fig. 16 is a diagram showing Compound (B-61) to Compound (B-72) as favorable specific examples of a compound represented by the chemical formula (Host-B).
[Fig. 17] Fig. 17 is a diagram showing Compound (B-73) to Compound (B-76) as favorable specific examples of a compound represented by the chemical formula (Host-B).
[Fig. 18] Fig. 18 is a diagram showing Compound (B-1) to Compound (3-1) to Compound (3-12) as favorable specific examples of a compound (metal complex) represented by the chemical formula (3).
[Fig. 19] Fig. 19 is a diagram showing Compound (B-1) to Compound (3-13) to Compound (3-24) as favorable specific examples of a compound (metal complex) represented by the chemical formula (3).
[Fig. 20] Fig. 20 is a diagram showing Compound (B-1) to Compound (3-25) to Compound (3-29) and Compound (3-31) to Compound (3-33) as favorable specific examples of a compound (metal complex) represented by the chemical formula (3).
[Fig. 21] Fig. 21 is a diagram showing Compound (4-1) to Compound (4-12) as favorable specific examples of a compound having a pyrimidine structure, the compound being represented by the general formula (4).
[Fig. 22] Fig. 22 is a diagram showing Compound (4-13) to Compound (4-24) as favorable specific examples of a compound having a pyrimidine structure, the compound being represented by the general formula (4).
[Fig. 23] Fig. 23 is a diagram showing Compound (4-25) to Compound (4-36) as favorable specific examples of a compound having a pyrimidine structure, the compound being represented by the general formula (4).
[Fig. 24] Fig. 24 is a diagram showing Compound (4-37) to Compound (4-48) as favorable specific examples of a compound having a pyrimidine structure, the compound being represented by the general formula (4).
[Fig. 25] Fig. 25 is a diagram showing Compound (4-49) to Compound (4-60) as favorable specific examples of a compound having a pyrimidine structure, the compound being represented by the general formula (4).
[Fig. 26] Fig. 26 is a diagram showing Compound (4-61) to Compound (4-69) as favorable specific examples of a compound having a pyrimidine structure, the compound being represented by the general formula (4).
[Fig. 27] Fig. 27 is a diagram showing Compound (4-70) to Compound (4-78) as favorable specific examples of a compound having a pyrimidine structure, the compound being represented by the general formula (4).
[Fig. 28] Fig. 28 is a diagram showing Compound (6-1) to Compound (6-12) as favorable specific examples of a compound having a benzoazole structure, the compound being represented by the general formula (6).
[Fig. 29] Fig. 29 is a diagram showing Compound (6-13) to Compound (6-24) as favorable specific examples of a compound having a benzoazole structure, the compound being represented by the general formula (6).
[Fig. 30] Fig. 30 is a diagram showing Compound (6-25) to Compound (6-36) as favorable specific examples of a compound having a benzoazole structure, the compound being represented by the general formula (6).
[Fig. 31] Fig. 31 is a diagram showing Compound (6-37) to Compound (6-48) as favorable specific examples of a compound having a benzoazole structure, the compound being represented by the general formula (6) .
[Fig. 32] Fig. 32 is a diagram showing Compound (6-49) to Compound (6-60) as favorable specific examples of a compound having a benzoazole structure, the compound being represented by the general formula (6).
[Fig. 33] Fig. 33 is a diagram showing Compound (6-61) to Compound (6-72) as favorable specific examples of a compound having a benzoazole structure, the compound being represented by the general formula (6).
[Fig. 34] Fig. 34 is a diagram showing Compound (6-73) to Compound (6-77) as favorable specific examples of a compound having a benzoazole structure, the compound being represented by the general formula (6).
[Fig. 35] Fig. 35 is a diagram showing Compound (7-1) to Compound (7-12) as favorable specific examples of a triphenylamine derivative represented by the general formula (7).
[Fig. 36] Fig. 36 is a diagram showing Compound (7-13) to Compound (7-24) as favorable specific examples of a triphenylamine derivative represented by the general formula (7).
[Fig. 37] Fig. 37 is a diagram showing Compound (7-25) to Compound (7-32) as favorable specific examples of a triphenylamine derivative represented by the general formula (7).
[Fig. 38] Fig. 38 is a diagram showing Compound (8-1) to Compound (8-8) as favorable specific examples of a triphenylamine derivative represented by the general formula (8).
[Fig. 39] Fig. 39 is a diagram showing Compound (8-9) to Compound (8-16) as favorable specific examples of a triphenylamine derivative represented by the general formula (8).
[Fig. 40] Fig. 40 is a diagram showing a configuration each of EL devices according to Examples 10 to 17 and Comparative Examples 1 to 4.
[Fig. 41] Fig. 41 is a 1H-NMR chart of Compound (1-1) according to Example 1 of the present invention.
[Fig. 42] Fig. 42 is a 1H-NMR chart of Compound (1-2) according to Example 2 of the present invention.
[Fig. 43] Fig. 43 is a 1H-NMR chart of Compound (1-3) according to Example 3 of the present invention.
[Fig. 44] Fig. 44 is a 1H-NMR chart of Compound (1-4) according to Example 4 of the present invention.
[Fig. 45] Fig. 45 is a 1H-NMR chart of Compound (1-5) according to Example 5 of the present invention.
[Fig. 46] Fig. 46 is a 1H-NMR chart of Compound (1-6) according to Example 6 of the present invention.
[Fig. 47] Fig. 47 is a 1H-NMR chart of Compound (1-20) according to Example 7 of the present invention. Mode(s) for Carrying Out the Invention

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₁ to R₁₈ in the general formula (1) to (2) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group. Further, these groups may form a ring with a single bond or may be bonded to each other via a substituted or unsubstituted methylene group, an oxygen atom, or a sulfur atom.

Specific examples of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the general formulae (1) to (2) include a deuterium atom, a cyano group, a nitro group; a halogen atom such as a fluorine atom, a chlorine atom, a bromine atom, and an iodine atom; a linear or branched alkoxy group having 1 to 6 carbon atoms such as a methoxy group, an ethoxy group, and a propyloxy group; an alkenyl group such as an allyl group; an aryloxy group such as a phenoxy group and a tolyloxy group; an arylalkoxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthryl group, a fluorenyl group, an indenyl group, a pyrenyl group, a perylenyl group, a fluoranthenyl group, and a triphenylenyl group; and an aromatic heterocyclic group such as a pyridyl group, a furanyl group, a pyranyl group, a thienyl group, a furil group, a pyrrolyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzimidazolyl group, a pyrazolyl group, a dibenzofuranyl group, a dibenzothiophenyl group, and a carbolinyl group. These substituted groups may be further substituted with other substituted groups. Further, these substituted groups may form a ring with a single bond, or may be bonded to each other via a substituted or unsubstituted methylene group, an oxygen atom, or a sulfur atom to form a ring.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by R₁ to R₁₈ in the general formulae (1) to (2) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. Further, these groups may form a ring with a single bond, or may be bonded to each other via a substituted or unsubstituted methylene group, an oxygen atom, or a sulfur atom to form a ring.

Specific examples of the "substituted group" in the "linear or branched alkyloxy group having 1 to 6 carbon atoms which has a substituted group" or "cycloalkyloxy group having 5 to 10 carbon atoms which has a substituted group" represented by R₁ to R₁₈ in the general formulae (1) to (2) include a deuterium atom, a cyano group, a nitro group; a halogen atom such as a fluorine atom, a chlorine atom, a bromine atom, and an iodine atom; a linear or branched alkoxy group having 1 to 6 carbon atoms such as a methoxy group, an ethoxy group, and a propyloxy group; an alkenyl group such as an allyl group; an aryloxy group such as a phenoxy group and a tolyloxy group; an arylalkoxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthryl group, a fluorenyl group, an indenyl group, a pyrenyl group, a perylenyl group, a fluoranthenyl group, and a triphenylenyl group; and an aromatic heterocyclic group such as a pyridyl group, a furanyl group, a pyranyl group, a thienyl group, a furil group, a pyrrolyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzimidazolyl group, a pyrazolyl group, a dibenzofuranyl group, a dibenzothiophenyl group, and a carbolinyl group. These substituted groups may be further substituted with other substituted groups. Further, these substituted groups may form a ring with a single bond, or may be bonded to each other via a substituted or unsubstituted methylene group, an oxygen atom, or a sulfur atom to form a ring.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₁ to R₁₈ and Ar₁ to Ar₄ in the general formulae (1) to (2) include a phenyl group, a biphenylyl group, a terphenylyl group, a naphthyl group, an anthryl group, a phenanthryl group, a fluorenyl group, an indenyl group, a pyrenyl group, a perylenyl group, a fluoranthenyl group, a triphenylenyl group, a pyridyl group, a furanyl group, a pyranyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalyl group, a benzimidazolyl group, a pyrazolyl group, a dibenzofuranyl group, a dibenzothienyl group, and a carbolinyl group. Further, these groups may form a ring with a single bond, or may be bonded to each other via a substituted or unsubstituted methylene group, an oxygen atom, or a sulfur atom to form a ring. Further, as a bonding position of the "substituted or unsubstituted aromatic heterocyclic group" represented by R₁ to R₁₈ and Ar₁ to Ar₄ in the general formulae (1) to (2), it is favorable to bond to the carbon atom of the "aromatic heterocyclic group" from the viewpoint of stability and heat resistance.

Specific examples of the "substituted group" in the "substituted aromatic hydrocarbon group", "substituted aromatic heterocyclic group", or "substituted fused polycyclic aromatic group" represented by R₁ to R₁₈ and Ar₁ to Ar₄ in the general formulae (1) to (2) include a deuterium atom, a trifluoromethyl group, a cyano group, a nitro group; a halogen atom such as a fluorine atom, a chlorine atom, a bromine atom, and an iodine atom; a linear or branched alkyl group having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; a linear or branched alkoxy group having 1 to 6 carbon atoms such as a methoxy group, an ethoxy group, and a propyloxy group; an alkenyl group such as an allyl group; an aralkyl group such as a benzyl group, a naphthylmethyl group, and a phenethyl group; an aryloxy group such as a phenoxy group and a tolyloxy group; an arylalkoxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthryl group, a fluorenyl group, an indenyl group, a pyrenyl group, a perylenyl group, a fluoranthenyl group, and a triphenylenyl group; an aromatic heterocyclic group such as a pyridyl group, a furanyl group, a pyranyl group, a thienyl group, a furil group, a pyrrolyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzimidazolyl group, a pyrazolyl group, a dibenzofuranyl group, a dibenzothiophenyl group, and a carbolinyl group; an arylvinyl group such as a styryl group and a naphthylvinyl group; an acyl group such as an acetyl group and a benzoyl group; a dialkylamino group such as a dimethylamino group and a diethylamino group; a disubstituted amino group substituted with an aromatic hydrocarbon group or a fused polycyclic aromatic group, such as a diphenylamino group and a dinaphthylamino group; a diaralkylamino group such as a dibenzylamino group and a diphenethylamino group; a disubstituted amino group substituted with an aromatic heterocyclic group, such as a dipyridylamino group and a dithienylamino group; a dialkenylamino group such as a diallylamino group; and a disubstituted amino group substituted with a substituted group selected from the group consisting of an alkyl group, an aromatic hydrocarbon group, a fused polycyclic aromatic group, an aralkyl group, an aromatic heterocyclic group, and an alkenyl group. These substituted groups may be further substituted. Further, these substituted groups may form a ring with a single bond, or may be bonded to each other via a substituted or unsubstituted methylene group, an oxygen atom, or a sulfur atom to form a ring.

Specific examples of the "aryloxy group" in the "substituted or unsubstituted aryloxy group" represented by R₁ to R₁₈ in the general formulae (1) to (2) include a phenoxy group, a biphenylyloxy group, a terphenylyloxy group, a naphthyloxy group, an anthryloxy group, a phenanthryloxy group, a fluorenyloxy group, an indenyloxy group, a pyrenyloxy group, and a perylenyloxy group. Further, these groups may form a ring with a single bond, or may be bonded to each other via a substituted or unsubstituted methylene group, an oxygen atom, or a sulfur atom to form a ring.

Specific examples of the "substituted group" in the "substituted aryloxy group" represented by R₁ to R₁₈ in the general formulae (1) to (2) include a deuterium atom, a trifluoromethyl group, a cyano group, a nitro group; a halogen atom such as a fluorine atom, a chlorine atom, a bromine atom, and an iodine atom; a linear or branched alkyl group having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; a linear or branched alkoxy group having 1 to 6 carbon atoms such as a methoxy group, an ethoxy group, and a propyloxy group; an alkenyl group such as an allyl group; an aralkyl group such as a benzyl group, a naphthylmethyl group, and a phenethyl group; an aryloxy group such as a phenoxy group and a tolyloxy group; an arylalkoxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthryl group, a fluorenyl group, an indenyl group, a pyrenyl group, a perylenyl group, a fluoranthenyl group, and a triphenylenyl group; an aromatic heterocyclic group such as a pyridyl group, a furanyl group, a pyranyl group, a thienyl group, a furil group, a pyrrolyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzimidazolyl group, a pyrazolyl group, a dibenzofuranyl group, a dibenzothiophenyl group, and a carbolinyl group; an arylvinyl group such as a styryl group and a naphthylvinyl group; an acyl group such as an acetyl group and a benzoyl group; a dialkylamino group such as a dimethylamino group and a diethylamino group; a disubstituted amino group substituted with an aromatic hydrocarbon group or a fused polycyclic aromatic group, such as a diphenylamino group and a dinaphthylamino group; a diaralkylamino group such as a dibenzylamino group and a diphenethylamino group; a disubstituted amino group substituted with an aromatic heterocyclic group, such as a dipyridylamino group and a dithienylamino group; a dialkenylamino group such as a diallylamino group; and a disubstituted amino group substituted with a substituted group selected from the group consisting of an alkyl group, an aromatic hydrocarbon group, a fused polycyclic aromatic group, an aralkyl group, an aromatic heterocyclic group, and an alkenyl group. These substituted groups may be further substituted. Further, these substituted groups may form a ring with a single bond, or may be bonded to each other via a substituted or unsubstituted methylene group, an oxygen atom, or a sulfur atom to form a ring.

Specific examples of the "divalent group of an aromatic hydrocarbon", "divalent group of an aromatic heterocycle", or "divalent group of a fused polycyclic aromatic" in the "divalent group of a substituted or unsubstituted aromatic hydrocarbon", "divalent group of a substituted or unsubstituted aromatic heterocycle", or "divalent group of a substituted or unsubstituted fused polycyclic aromatic" represented by A in the general formula (1) include a phenylene group, a biphenylene group, a terphenylene group, a tetrakisphenylene group, a naphthylene group, an anthrylene group, a phenanthrylene group, a fluorenylene group, a phenanthrolylene group, an indenylene group, a pyrenylene group, a perylenylene group, a fluoranthenylene group, a triphenylenylene group, a pyridinylene group, a pyrimidinylene group, a quinolylene group, an isoquinolylene group, an indolylene group, a carbazolylene group, a quinoxalylene group, a benzimidazolylene group, a pyrazolylene group, a naphthyridinylene group, a phenanthrolinylene group, an acridinylene group, a thiophenylene group, a benzothiophenylene group, a benzothiazolylene group, and a dibenzothiophenylene group. Further, these groups may form a ring with the "substituted or unsubstituted aromatic hydrocarbon group", "substituted or unsubstituted aromatic heterocyclic group", or "substituted or unsubstituted fused polycyclic aromatic group" represented by Ar₂ in the general formula (1), with a single bond, or may be bonded to each other via a substituted or unsubstituted methylene group, an oxygen atom, or a sulfur atom to form a ring.

Specific examples of the "substituted group" in the "divalent group of a substituted aromatic hydrocarbon", "divalent group of a substituted aromatic heterocycle", or "divalent group of a substituted fused polycyclic aromatic" represented by A in the general formula (1) include a deuterium atom, a cyano group, a nitro group; a halogen atom such as a fluorine atom, a chlorine atom, a bromine atom, and an iodine atom; a linear or branched alkyl group having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; a linear or branched alkoxy group having 1 to 6 carbon atoms such as a methoxy group, an ethoxy group, and a propyloxy group; an alkenyl group such as an allyl group; an aralkyl group such as a benzyl group, a naphthylmethyl group, and a phenethyl group; an aryloxy group such as a phenoxy group and a tolyloxy group; an arylalkoxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthryl group, a fluorenyl group, an indenyl group, a pyrenyl group, a perylenyl group, a fluoranthenyl group, and a triphenylenyl group; an aromatic heterocyclic group such as a pyridyl group, a furanyl group, a pyranyl group, a thienyl group, a furil group, a pyrrolyl group, a thiophenyl group, a quinolyl group, an isoquinolyl group, a benzofuranyl group, a benzothiophenyl group, an indolyl group, a carbazolyl group, a benzoxazolyl group, a benzothiazolyl group, a quinoxalyl group, a benzimidazolyl group, a pyrazolyl group, a dibenzofuranyl group, a dibenzothiophenyl group, and a carbolinyl group; an arylvinyl group such as a styryl group and a naphthylvinyl group; and an acyl group such as an acetyl group and a benzoyl group. These substituted groups may be further substituted. Further, these substituted groups may form a ring with a single bond, or may be bonded to each other via a substituted or unsubstituted methylene group, an oxygen atom, or a sulfur atom to form a ring.

Specific examples of the "substituted or unsubstituted alkyl group having 1 to 15 carbon atoms " represented by R₁₉ to R₂₈ and Ra in the general formula (HOST-A) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

Examples of the "substituted group" in the "substituted or unsubstituted alkyl group having 1 to 15 carbon atoms" represented by R₁₉ to R₂₈ and Ra in the general formula (HOST-A) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the above-mentioned general formulae (1) and (2), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the above-mentioned general formulae (1) and (2) can be taken.

Specific examples of the "substituted or unsubstituted aryl group having 6 to 12 ring carbon atoms" represented by R₁₉ to R₂₈ and Ra in the general formula (HOST-A) include a phenyl group, a biphenylyl group, a 1-naphthyl group, a 2-naphthyl group, a fluorophenyl group, a difluorophenyl group, a trifluorophenyl group, a tetrafluorophenyl group, a pentafluorophenyl group, a tolyl group, a nitrophenyl group, a cyanophenyl group, a fluorobiphenylyl group, a nitrobiphenylyl group, a cyanobiphenyl group, a cyanonaphthyl group, a nitronaphthyl group, and a fluoronaphthyl group. Of the above, a phenyl group or a biphenylyl group is particularly favorable.

Examples of the "substituted group" in the "substituted or unsubstituted aryl group having 6 to 12 ring carbon atoms" represented by R₁₉ to R₂₈ and Ra in the general formula (HOST-A) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "alkyl group having 1 to 15 carbon atoms" represented by R₂₉ to R₃₂ in the general formula (HOST-B) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

Examples of the "substituted group" in the "alkyl group having 1 to 15 carbon atoms" represented by R₂₉ to R₃₂ in the general formula (HOST-B) include the similar ones as described for the "substituted group" in the "linear or branched alkyl group having 1 to 6 carbon atoms which has a substituted group", "cycloalkyl group having 5 to 10 carbon atoms which has a substituted group ", or "linear or branched alkenyl group having 2 to 6 carbon atoms which has a substituted group" represented by R₁ to R₁₈ in the above-mentioned general formulae (1) and (2), and aspects similar to those of the "substituted group" in the "linear or branched alkyl group having 1 to 6 carbon atoms which has a substituted group", "cycloalkyl group having 5 to 10 carbon atoms which has a substituted group ", or "linear or branched alkenyl group having 2 to 6 carbon atoms which has a substituted group" represented by R₁ to R₁₈ in the above-mentioned general formulae (1) and (2) can be taken.

Specific examples of the "substituted or unsubstituted aryl group having 6 to 50 ring carbon atoms" or "substituted or unsubstituted heteroaryl group having 4 to 50 ring carbon atoms" represented by R₂₉ to R₃₂ in the general formula (HOST-B) include a phenyl group, a biphenyl group, a terphenyl group, a naphthyl group, an anthryl group, a phenanthryl group, a fluorenyl group, an indenyl group, a pyrenyl group, an acetonaphthenyl group, a fluoranthenyl group, a triphenylenyl group, a pyridyl group, a pyranyl group, a quinolyl group, an isoquinolyl group, a benzofuranyl group, a benzothienyl group, an indolyl group, a carbazolyl group, a benzoxazolyl group, a benzothiazolyl group, a quinoxalyl group, a benzimidazolyl group, a dibenzofuranyl group, and a dibenzothienyl group.

Examples of the "substituted group" in the "substituted or unsubstituted aryl group having 6 to 50 ring carbon atoms" or "substituted or unsubstituted heteroaryl group having 4 to 50 ring carbon atoms" represented by R₂₉ to R₃₂ in the general formula (HOST-B) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "substituted or unsubstituted arylene group having 6 to 30 ring carbon atoms" or "substituted or unsubstituted heteroarylene group having 5 to 30 ring carbon atoms" represented by Y in the general formula (HOST-B) include a phenylene group, a biphenylene group, a terphenylene group, a naphthylene group, an anthrylene group, a phenanthrylene group, a fluorenylene group, an indenylene group, a pyrenylene group, an acetonaphthenylene group, a fluoranthenylene group, a triphenylenylene group, a pyridylene group, a pyranylene group, a quinolylene group, an isoquinolylene group, a benzofuranylene group, a benzothienylene group, an indolylene group, a carbazolylene group, a benzoxazolylene group, a benzothiazolylene group, a quinoxalylene group, a benzimidazolylene group, a pyrazolylene group, a dibenzofuranylene group, and a dibenzothienylene group.

Examples of the "substituted group" in the "substituted or unsubstituted arylene group having 6 to 30 ring carbon atoms" or "substituted or unsubstituted heteroarylene group having 5 to 30 ring carbon atoms" represented by Y in the general formula (HOST-B) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "substituted or unsubstituted aryl group having 6 to 30 ring carbon atoms" or "substituted or unsubstituted heteroaryl group having 5 to 30 ring carbon atoms" represented by Ar₅ in the general formula (HOST-B) include a phenyl group, a naphthyl group, an anthracenyl group, a phenanthryl group, a biphenyl group, a p-terphenyl group, an m-terphenyl group, a quarterphenyl group, a fluorenyl group, a triphenylene group, a biphenylene group, a pyrenyl group, a benzofluoranthenyl group, a chrysenyl group, a phenylnaphthyl group, a naphthylphenyl group, a pyridyl group, a quinolyl group, an isoquinolyl group, a benzofuryl group, a benzothienyl group, an indolyl group, a benzoxazolyl group, a benzothiazolyl group, a quinoxalyl group, a benzimidazolyl group, a dibenzofuryl group, a dibenzothienyl group, and a carbazolyl group.

Examples of the "substituted group" in the "substituted or unsubstituted aryl group having 6 to 30 ring carbon atoms" or "substituted or unsubstituted heteroaryl group having 5 to 30 ring carbon atoms" represented by Ar₅ in the general formula (HOST-B) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₃₃ to R₄₈ in the general formula (3) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group. Further, these groups may form a ring with a single bond, or may be bonded to each other via a substituted or unsubstituted methylene group, an oxygen atom, or a sulfur atom to form a ring.

Examples of the "substituted group" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₃₃ to R₄₈ in the general formula (3) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the above-mentioned general formulae (1) and (2), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the above-mentioned general formulae (1) and (2) can be taken.

Specific examples of the "aryloxy group" in the "substituted or unsubstituted aryloxy group" represented by R₃₃ to R₄₈ in the general formula (3) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group. These groups may form a ring with a single bond, or may be bonded to each other via a substituted or unsubstituted methylene group, an oxygen atom, or a sulfur atom to form a ring.

Examples of the "substituted group" in the "substituted or unsubstituted aryloxy group" represented by R₃₃ to R₄₈ in the general formula (3) include the similar ones as described for the "substituted group" in the "substituted or unsubstituted aryloxy group" represented by R₁ to R₁₈ in the above-mentioned general formulae (1) and (2), and aspects similar to those of the "substituted group" in the "substituted or unsubstituted aryloxy group" represented by R₁ to R₁₈ in the above-mentioned general formulae (1) and (2) can be taken.

The "substituted or unsubstituted aromatic hydrocarbon group", "substituted or unsubstituted aromatic heterocyclic group", "substituted or unsubstituted fused polycyclic aromatic group", or "substituted or unsubstituted aryloxy group" represented by R₃₃ to R₄₈ in the general formula (3) can specifically be, but not limited to, a phenyl group, a naphthyl group, an anthracenyl group, a phenanthryl group, a naphthacenyl group, a pyrenyl group, a biphenylyl group, a p-terphenyl group, an m-terphenyl group, a chrysenyl group, a triphenylenyl group, a perylenyl group, an indenyl group, a furanyl group, a thiophenyl group, a pyrrolyl group, a pyrazolyl group, an imidazolyl group, a triazolyl group, an oxazolyl group, a thiazolyl group, an oxadiazolyl group, a thiadiazolyl group, a pyridyl group, a pyrimidinyl group, a pyrazinyl group, a triazinyl group, a benzofuranyl group, a benzothiophenyl group, a benzimidazolyl group, an indolyl group, a quinolinyl group, an isoquinolinyl group, a quinazolinyl group, a quinoxalinyl group, a naphthyridinyl group, a benzoxazinyl group, a benzthiazinyl group, an acridinyl group, a phenazinyl group, a phenothiazinyl group, a phenoxazinyl group, or a combination thereof.

Examples of the "substituted group" in the "substituted or unsubstituted aromatic hydrocarbon group", "substituted or unsubstituted aromatic heterocyclic group", "substituted or unsubstituted fused polycyclic aromatic group", or "substituted or unsubstituted aryloxy group" represented by R₃₃ to R₄₈ in the general formula (3) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "aromatic hydrocarbon group" or "fused polycyclic aromatic group" in the "substituted or unsubstituted aromatic hydrocarbon group" or "substituted or unsubstituted fused polycyclic aromatic group" represented by Ar₆ to Ar₈ in the general formula (4) include a phenyl group, a biphenylyl group, a terphenylyl group, a tetrakisphenyl group, a styryl group, a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group.

Examples of the "substituted group" in the "substituted aromatic hydrocarbon group" or "substituted fused polycyclic aromatic group" represented by Ar₆ to Ar₈ in the general formula (4) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "aromatic heterocyclic group" in the "substituted or unsubstituted aromatic heterocyclic group" represented by Ar₉ in the structural formula (5) include a triazinyl group, a pyridyl group, a pyrimidinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Examples of the "substituted group" in the "substituted aromatic heterocyclic group" represented by Ar₉ in the structural formula (5) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "linear or branched alkyl group having 1 to 6 carbon atoms" represented by R₄₉ to R₅₂ in the structural formula (5) include a methyl group, an ethyl group, an n-propyl group, an i-propyl group, an n-butyl group, a 2-methylpropyl group, a t-butyl group, an n-pentyl group, a 3-methylbutyl group, a tert-pentyl group, an n-hexyl group, an iso-hexyl group, and a tert-hexyl group.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₄₉ to R₅₂ in the structural formula (5) include a phenyl group, a biphenylyl group, a terphenylyl group, a tetrakisphenyl group, a styryl group, a naphthyl group, an anthracenyl group, an acenaphthenyl group, a phenanthrenyl group, a fluorenyl group, an indenyl group, a pyrenyl group, a perylenyl group, a fluoranthenyl group, a triphenylenyl group, a triazinyl group, a pyridyl group, a pyrimidinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Examples of the " substituted group" in the "substituted aromatic hydrocarbon group", "substituted aromatic heterocyclic group", or "substituted fused polycyclic aromatic group" represented by R₄₉ to R₅₂ in the structural formula (5) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

As Ar₆ in the general formula (4), a phenyl group, a biphenylyl group, a naphthyl group, an anthracenyl group, an acenaphthenyl group, a phenanthrenyl group, a fluorenyl group, an indenyl group, a pyrenyl group, a perylenyl group, a fluoranthenyl group, or a triphenylenyl group is favorable, and a phenyl group, a biphenylyl group, a naphthyl group, an anthracenyl group, a phenanthrenyl group, a pyrenyl group, a fluoranthenyl group, or a triphenylenyl group is more favorable. Here, the phenyl group favorably has a substituted or unsubstituted fused polycyclic aromatic group as a substituted group, and more favorably has a substituted group selected from the group consisting of a naphthyl group, an anthracenyl group, a phenanthrenyl group, a pyrenyl group, a fluoranthenyl group, and a triphenylenyl group.

As Ar₇ in the general formula (4), a phenyl group having a substituted group is favorable. As the substituted group in this case, an aromatic hydrocarbon group such as a phenyl group, a biphenylyl group, and a terphenyl group, or a fused polycyclic aromatic group such as a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group is favorable, and a phenyl group, a naphthyl group, an anthracenyl group, a phenanthrenyl group, a pyrenyl group, a fluoranthenyl group, or a triphenylenyl group is more favorable.

As Ar₈ in the general formula (4), a phenyl group having a substituted group is favorable. As the substituted group in this case, an aromatic hydrocarbon group such as a phenyl group, a biphenylyl group, and a terphenyl group, or a fused polycyclic aromatic group such as a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group is favorable, and a phenyl group, a naphthyl group, an anthracenyl group, a phenanthrenyl group, a pyrenyl group, a fluoranthenyl group, or a triphenylenyl group is more favorable.

As Ar₉ in the structural formula (5), a nitrogen containing heterocyclic group such as a triazinyl group, a pyridyl group, a pyrimidinyl group, a pyrrolyl group, a quinolyl group, an isoquinolyl group, an indolyl group, a carbazolyl group, a benzoxazolyl group, a benzothiazolyl group, a quinoxalinyl group, a benzimidazolyl group, a pyrazolyl group, a naphthyridinyl group, a phenanthrolinyl group, an acridinyl group, or a carbolinyl group is favorable, a triazinyl group, a pyridyl group, a pyrimidinyl group, a quinolyl group, an isoquinolyl group, an indolyl group, a quinoxalinyl group, a benzimidazolyl group, a naphthyridinyl group, a phenanthrolinyl group, or an acridinyl group is more favorable, and a pyridyl group, a pyrimidinyl group, a quinolyl group, an isoquinolyl group, an indolyl group, a quinoxalinyl group, a benzimidazolyl group, a phenanthrolinyl group, or an acridinyl group is particularly favorable.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by AR₁₀, AR₁₁, and V₁ in the general formula (6) include a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Examples of the "substituted group" in the "substituted or unsubstituted aromatic hydrocarbon group", "substituted or unsubstituted aromatic heterocyclic group", or "substituted or unsubstituted fused polycyclic aromatic group" represented by Ar₉, AR₁₀, AR₁₁, and V₁ in the structural formula (6) include the similar ones as described for the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2), and aspects similar to those of the "substituted group" in the "substituted aromatic hydrocarbon group", "substituted aromatic heterocyclic group", or "substituted fused polycyclic aromatic group" represented by Ar₁ to Ar₄ in the above-mentioned general formulae (1) and (2) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", , or "linear or branched alkenyl group having 2 to 6 carbon atoms which may have a substituted group" represented by V₁ in the general formula (6) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group.

Examples of the "substituted group" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by V₁ in the general formula (6) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the above-mentioned general formulae (1) and (2), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₁ to R₁₈ in the above-mentioned general formulae (1) and (2) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₅₃ to R₅₈ in the general formula (7) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group. These groups may form a ring with a single bond, or may be bonded to each other via a substituted or unsubstituted methylene group, an oxygen atom, or a sulfur atom to form a ring.

Specific examples of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₅₃ to R₅₈ in the general formula (7) include a deuterium atom, a cyano group, a nitro group; a halogen atom such as a fluorine atom, a chlorine atom, a bromine atom, and an iodine atom; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; and an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group. These substituted groups may be further substituted with the exemplified substituted groups. Further, these substituted groups may form a ring with a single bond, or may be bonded to each other via a substituted or unsubstituted methylene group, an oxygen atom, or a sulfur atom to form a ring.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by R₅₃ to R₅₈ in the general formula (7) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. These groups may form a ring with a single bond, or may be bonded to each other via a substituted or unsubstituted methylene group, an oxygen atom, or a sulfur atom to form a ring.

Further, these groups may each have a substituted group. Examples of such a substituted group include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₅₃ to R₅₈ in the above-mentioned general formula (7), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₅₃ to R₅₈ in the above-mentioned general formula (7) can be taken.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₅₃ to R₅₈ in the general formula (7) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group. These groups may form a ring with a single bond, or may be bonded to each other via a substituted or unsubstituted methylene group, an oxygen atom, or a sulfur atom to form a ring.

Specific examples of the "substituted group" in the "substituted or unsubstituted aromatic hydrocarbon group", "substituted or unsubstituted aromatic heterocyclic group", or "substituted or unsubstituted fused polycyclic aromatic group" represented by R₅₃ to R₅₈ in the general formula (7) include a deuterium atom, a cyano group, a nitro group; a halogen atom such as a fluorine atom, a chlorine atom, a bromine atom, and an iodine atom; a linear or branched alkyl group having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; an arylvinyl group such as a styryl group and a naphthylvinyl group; an acyl group such as an acetyl group and a benzoyl group; and a silyl group such as a trimethylsilyl group and a triphenylsilyl group. These substituted groups may be further substituted with the exemplified substituted groups. Further, these substituted groups may form a ring with a single bond, or may be bonded to each other via a substituted or unsubstituted methylene group, an oxygen atom, or a sulfur atom to form a ring.

Specific examples of the "aryloxy group" in the "substituted or unsubstituted aryloxy group" represented by R₅₃ to R₅₈ in the general formula (7) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group. These groups may form a ring with a single bond, or may be bonded to each other via a substituted or unsubstituted methylene group, an oxygen atom, or a sulfur atom to form a ring.

Examples of the "substituted group" in the "substituted or unsubstituted aryloxy group" represented by R₅₃ to R₅₈ in the general formula (7) include the similar ones as described for the "substituted group" in the "substituted or unsubstituted aryloxy group" represented by R₁ to R₁₈ in the above-mentioned general formulae (1) and (2), and aspects similar to those of the "substituted group" in the "substituted or unsubstituted aryloxy group" represented by R₁ to R₁₈ in the above-mentioned general formulae (1) and (2) can be taken.

In the general formula (7), r₁ to r₆ may be the same as or different from each other, r₁, r₂, r₅, and r₆ each represent an integer of 0 to 5, and r₃ and r₄ each represent an integer of 0 to 4. In the case where r₁, r₂, r₅, or r₆ is an integer of 2 to 5, or in the case where r₃ or r₄ is an integer of 2 to 4, a plurality of R₅₃, a plurality of R₅₄, a plurality of R₅₅, a plurality of R₅₆, a plurality of R₅₇, or a plurality of R₅₈ bonded to the same benzene ring may be the same as or different from each other, may form a ring with a single bond, or may be bonded to each other via a substituted or unsubstituted methylene group, an oxygen atom, or a sulfur atom to form a ring.

Examples of the "divalent linking group" represented by K₁ in the general formula (7) include a divalent group such as a "linear or branched alkylene group having 1 to 6 carbon atoms" such as a methylene group, an ethylene group, an n-propylene group, an isopropylene group, an n-butylylene group, an isobutylene group, a tert-butylylene group, an n-pentylylene group, an isopentylylene group, an neopentylylene group, and an n-hexylylene group; a "cycloalkylene group having 5 to 10 carbon atoms" such as a cyclopentylylene group, a cyclohexylylene group, and an adamantylylene group; a "linear or branched alkenylene group having 2 to 6 carbon atoms" such as a vinylene group, an arylene group, an isopropenylene group, and a butenylene group; a "divalent group of an aromatic hydrocarbon" formed by removing two hydrogen atoms from an aromatic hydrocarbon such as benzene, biphenyl, terphenyl, and tetrakisphenyl; and a "divalent group of a fused polycyclic aromatic" formed by removing two hydrogen atoms from a fused polycyclic aromatic such as naphthalene, anthracene, acenaphthalene, fluorene, phenanthrene, indan, pyrene, and triphenylene.

Further, these divalent groups may each have a substituted group. Examples of the substituted group of the "linear or branched alkylene group having 1 to 6 carbon atoms", "cycloalkylene group having 5 to 10 carbon atoms", or "linear or branched alkenylene group having 2 to 6 carbon atoms" include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by R₅₃ to R₅₈ in the above-mentioned general formula (7), examples of the substituted group of the "divalent group of an aromatic hydrocarbon" or "divalent group of a fused polycyclic aromatic" include the similar ones as described for the "substituted group" in the "substituted or unsubstituted aromatic hydrocarbon group", "substituted or unsubstituted aromatic heterocyclic group", or "substituted or unsubstituted fused polycyclic aromatic group" represented by R₅₃ to R₅₈ in the above-mentioned general formula (7), and aspects similar to those of these substituted groups can be taken.

Examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₅₉ to R₇₀ in the general formula (8) include the similar ones as described for the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₅₃ to R₅₈ in the above-mentioned general formula (7), and aspects similar to those of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by R₅₃ to R₅₈ in the above-mentioned general formula (7) can be taken.

Examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by R₅₉ to R₇₀ in the general formula (8) include the similar ones as described for the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by R₅₃ to R₅₈ the above-mentioned general formula (7), and aspects similar to those of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by R₅₃ to R₅₈ the above-mentioned general formula (7) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₅₉ to R₇₀ in the general formula (8) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₅₃ to R₅₈ in the above-mentioned general formula (7). These groups may form a ring with a single bond, or may be bonded to each other via a substituted or unsubstituted methylene group, an oxygen atom, or a sulfur atom to form a ring.

Further, these groups may each have a substituted group. Examples of such a substituted group include the similar ones as described for the "substituted group" in the "substituted aromatic hydrocarbon group", "substituted aromatic heterocyclic group", or substituted fused polycyclic aromatic group" represented by R₅₃ to R₅₈ in the above-mentioned general formula (7), and aspects similar to those of the "substituted group" in the "substituted aromatic hydrocarbon group", "substituted aromatic heterocyclic group", or substituted fused polycyclic aromatic group" represented by R₅₃ to R₅₈ in the above-mentioned general formula (7) can be taken.

Examples of the "aryloxy group" in the "substituted or unsubstituted aryloxy group" represented by R₅₉ to R₇₀ in the general formula (8) include the similar ones as described for the "aryloxy group" in the "substituted or unsubstituted aryloxy group" represented by R₅₃ to R₅₈ in the above-mentioned general formula (7), and aspects similar to those of the "aryloxy group" in the "substituted or unsubstituted aryloxy group" represented by R₅₃ to R₅₈ in the above-mentioned general formula (7) can be taken.

In the general formula (8), r₇ to r₁₈ may be the same as or different from each other, r₇ to r₁₂ each represent an integer of 0 to 5, and r₁₃ to r₁₈ each represent an integer of 0 to 4. In the case where any of r₇ to r₁₂ is an integer of 2 to 5, or in the case where any of r₁₃ to r₁₈ is an integer of 2 to 4, a plurality of R₅₉, a plurality of R₆₀, a plurality of R₆₁, a plurality of R₆₂, a plurality of R₆₃, a plurality of R₆₄, a plurality of R₆₅, a plurality of R₆₆, a plurality of R₆₇, a plurality of R₆₈, a plurality of R₆₉, or a plurality of R₇₀ bonded to the same benzene ring may be the same as or different from each other, may form a ring with a single bond, or may be bonded to each other via a substituted or unsubstituted methylene group, an oxygen atom, or a sulfur atom to form a ring.

Examples of the "divalent linking group" represented by K₂, K₃, and K₄ in the general formula (8) include the similar ones as described for the "divalent linking group" represented by K₁ in the above-mentioned general formula (7), and aspects similar to those of the "divalent linking group" represented by K₁ in the above-mentioned general formula (7) can be taken.

The compound having an indenocarbazole ring structure according to the present invention, which is represented by the general formula (1), is a novel compound, and has excellent electron blocking performance, an excellent amorphous property, and high stability in a thin-film state as compared with the existing hole transport material.

The compound having an indenocarbazole ring structure according to the present invention, which is represented by the general formula (1), can be used as a host material of a second hole transport layer adjacent to a light-emitting layer of an organic EL device and/or the light-emitting layer. Since a material having a high hole injection property, a high mobility of holes, a high electron blocking property, and high stability for electrons as compared with the existing material is used, there are provided effects of being capable of confining excitons generated in the light-emitting layer, improving the probability of recombination of holes and electrons, achieving high light emission efficiency, and improving the durability of the organic EL device because the drive voltage is reduced.

The compound having an indenocarbazole ring structure according to the present invention, which is represented by the general formula (1), can be used also as a constituent material of a light-emitting layer of an organic EL device. The compound has an excellent hole transport property as compared with the existing material, and provides an effect of more suitably improving the light emission efficiency of the organic EL device particularly in the case where it contains a green phosphorescent light-emitting material.

The organic EL device according to the present invention is capable of achieving high efficiency and a high durability because it uses a compound having an indenocarbazole ring structure, which has a high mobility of holes, excellent electron blocking performance, an excellent amorphous property, and high stability in a thin-film state as compared with the existing hole transport material.

The compound having an indenocarbazole ring structure according to the present invention is useful as a light-emitting layer of an organic EL device or as a second hole transport layer adjacent to the light-emitting layer, has excellent electron blocking performance, an excellent durability for electrons, and a favorable amorphous property, and is stable in a thin-film state and excellent in heat resistance. The organic EL device according to the present invention has high light emission efficiency and high power efficiency, and has an excellent durability for electrons, which makes it possible to prolong the device lifetime.

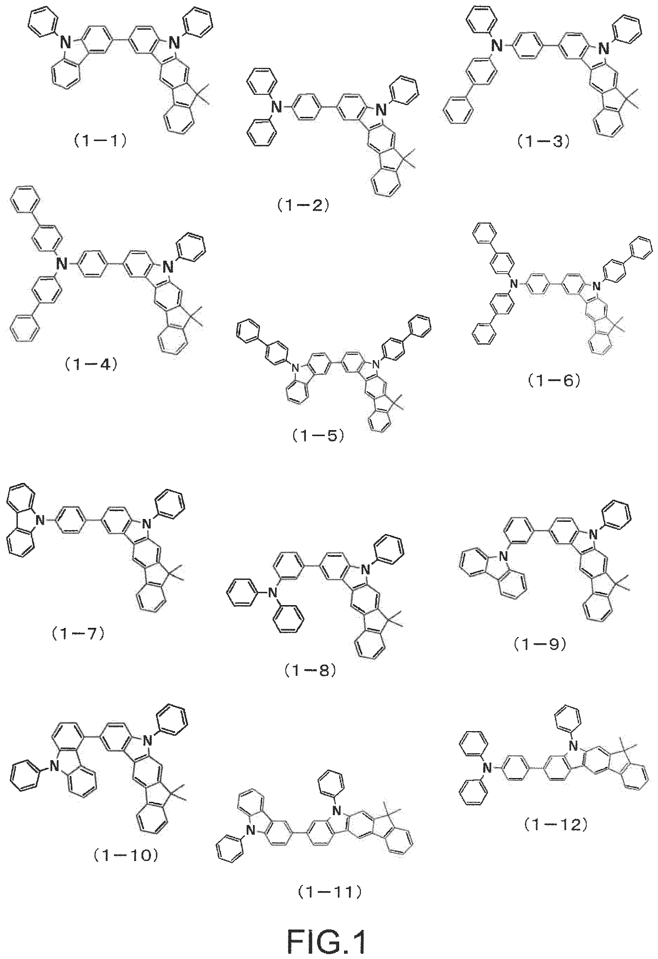

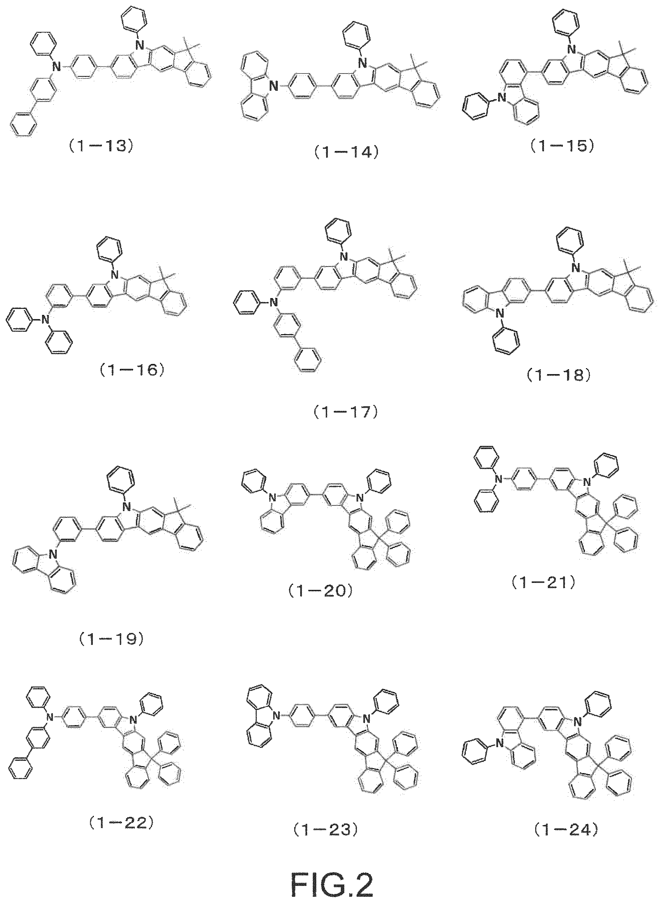

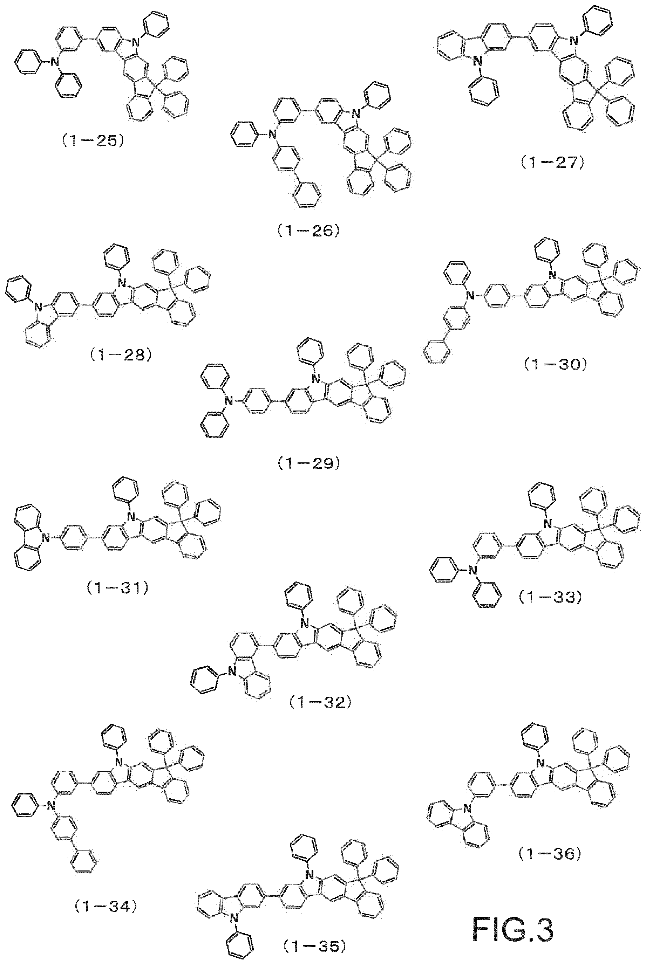

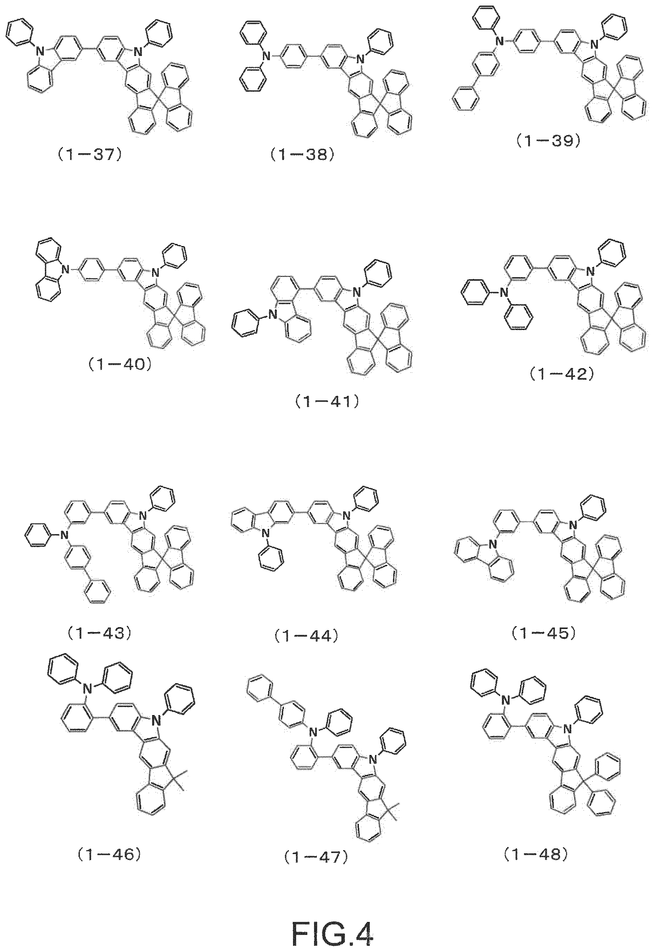

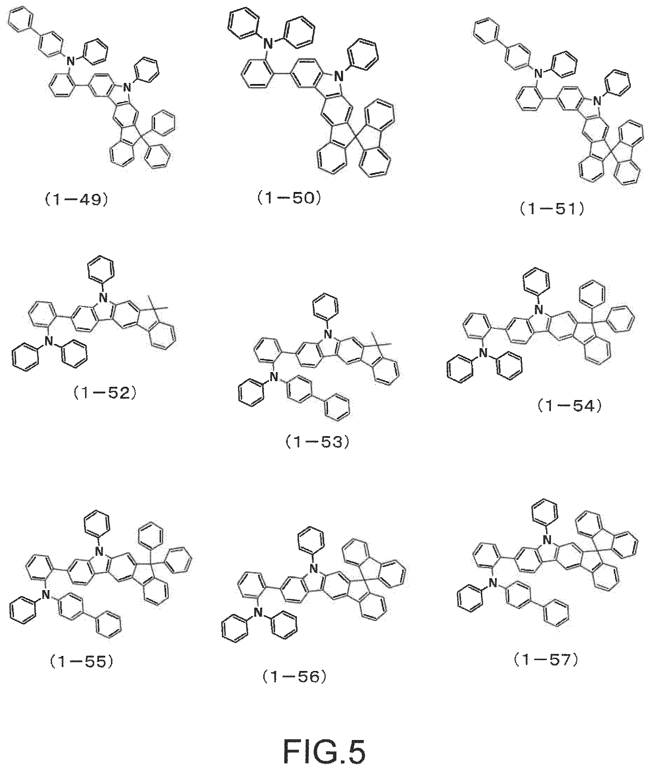

Compound (1-1) to Compound (1-57) are shown in Fig. 1 to Fig. 5 as specific examples of favorable compounds among the compounds having an indenocarbazole ring structure, which are represented by the general formula (1). However, the present invention is not limited to these Compounds.

Note that the above-mentioned indenocarbazole compound can be synthesized in accordance with a method known per se (see, for example, Patent Literature 8).

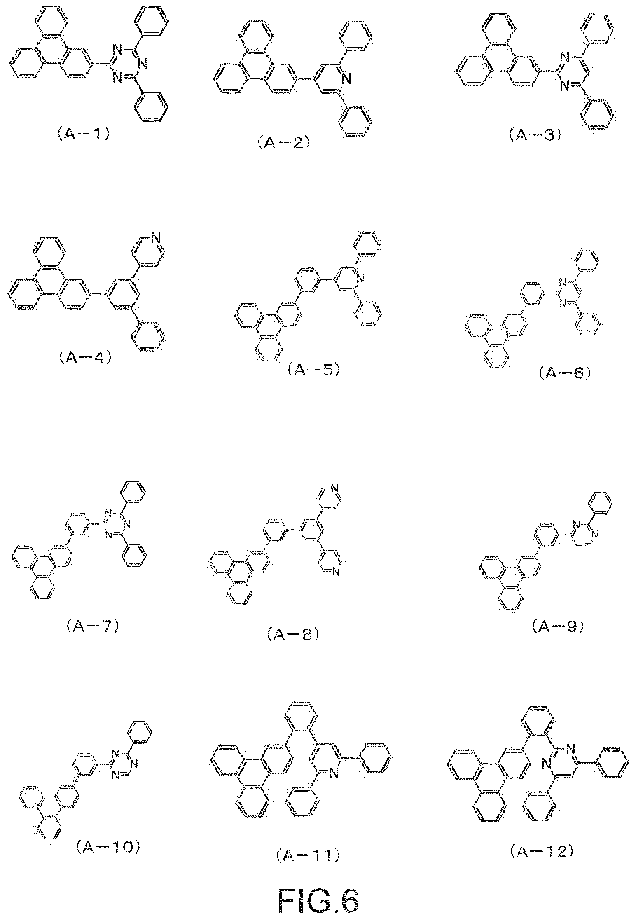

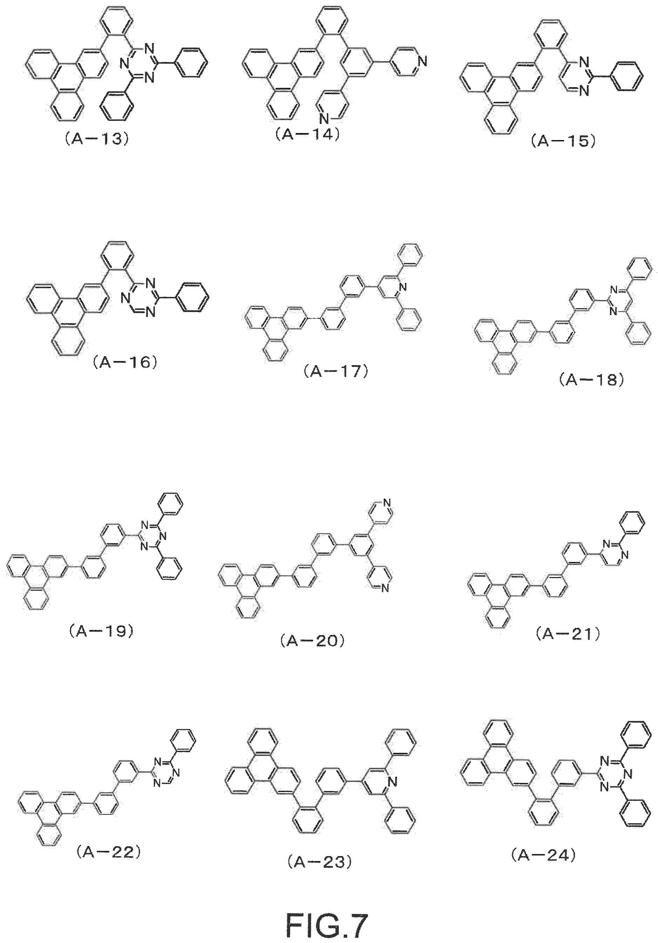

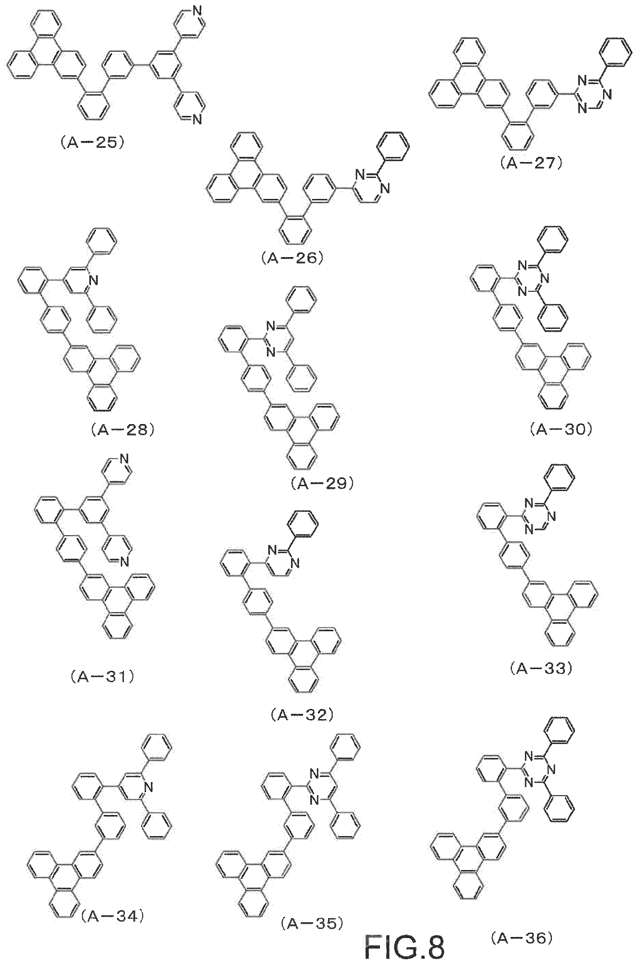

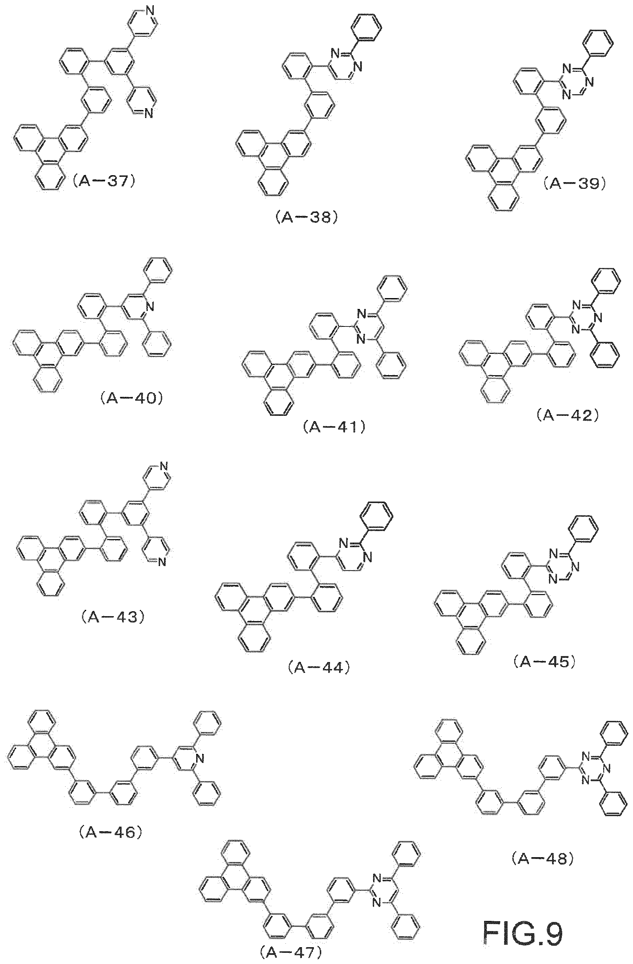

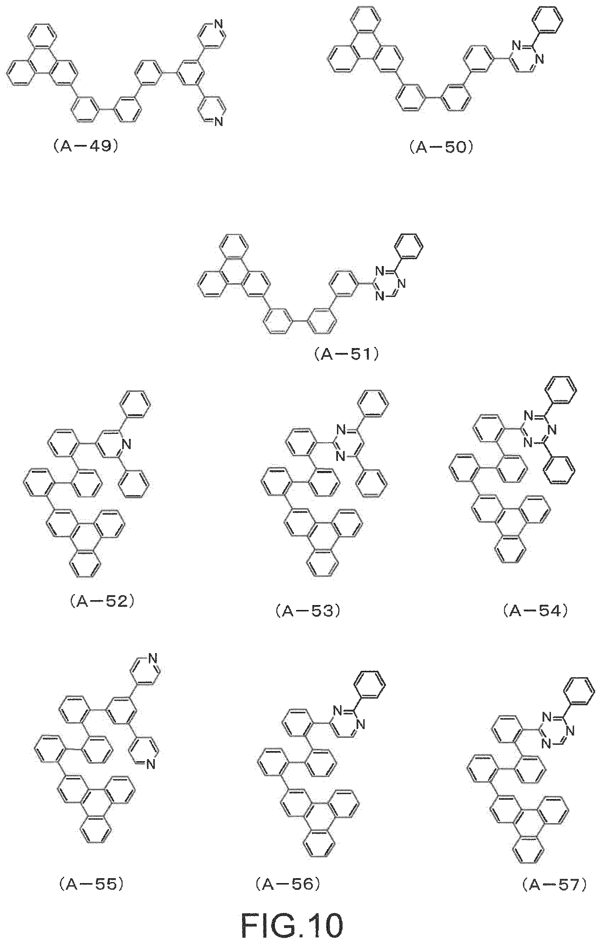

Compound (A-1) to Compound (A-57) are shown in fig. 6 to fig.10 as specific examples of favorable compounds among the compounds represented by the chemical formula (Host-A), which are suitably used for the organic EL device according to the present invention. However, the present invention is not limited to these Compounds.

Note that the above-mentioned compound having a nitrogen-containing heteroaromatic ring structure can be synthesized in accordance with a method known per se (see, for example, Patent Literatures 4 and 5).

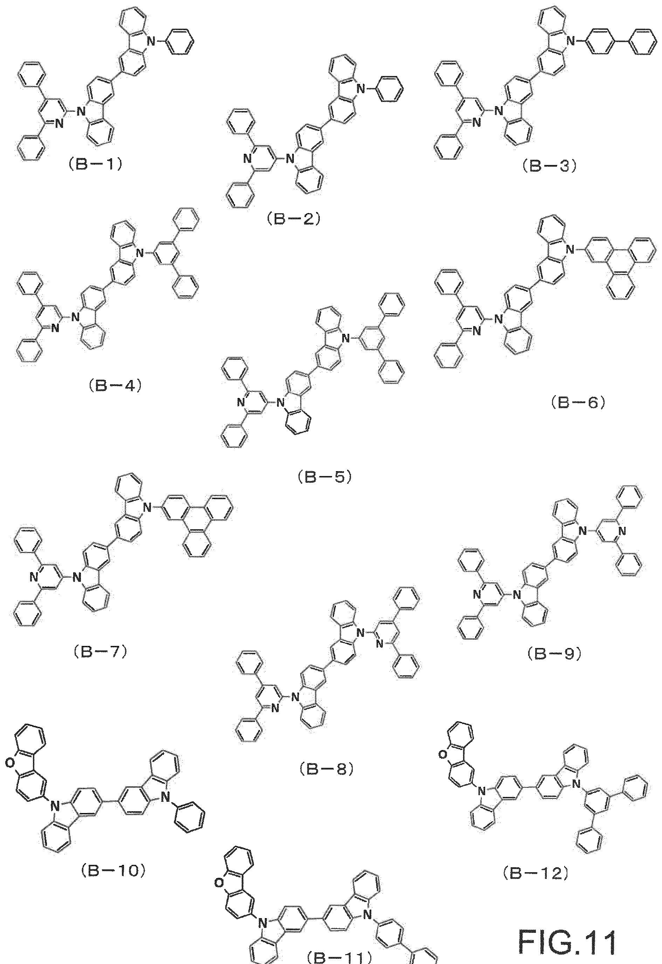

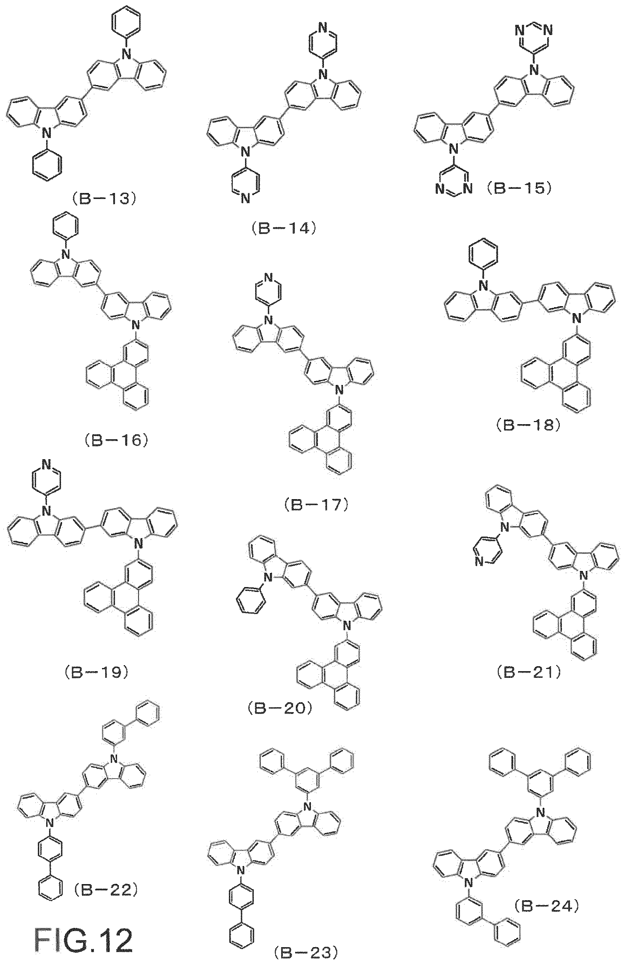

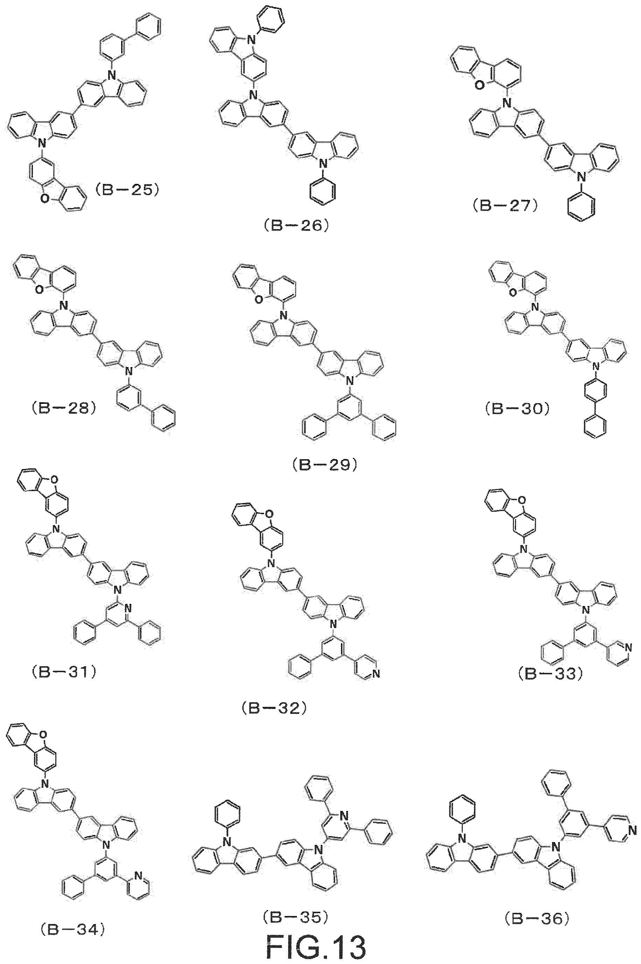

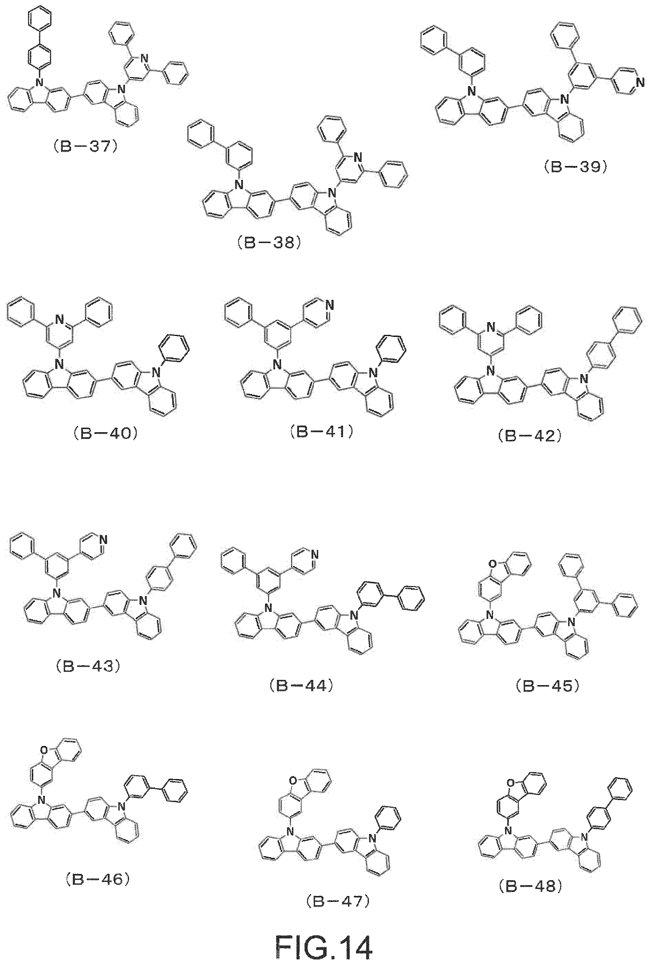

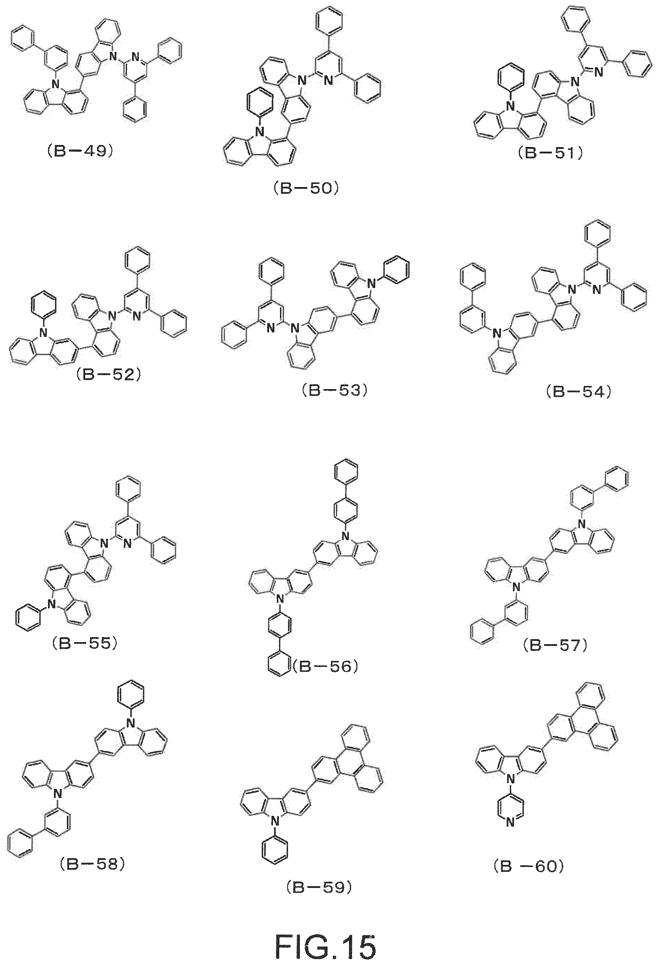

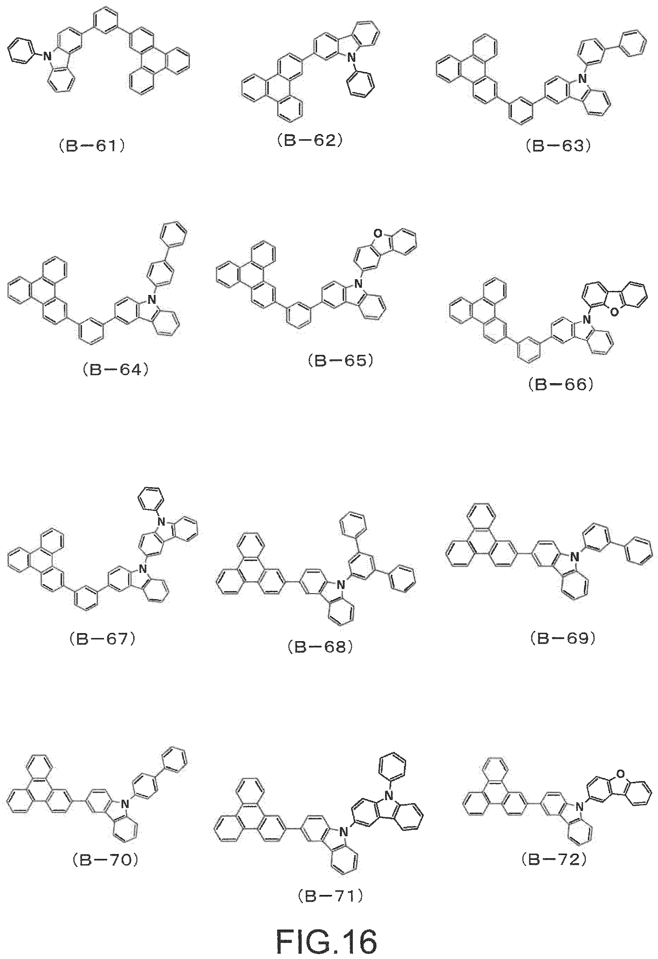

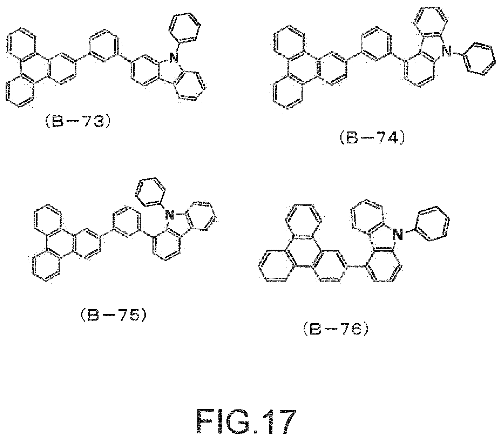

Compound (B-1) to Compound (B-76) are shown in Fig. 11 to Fig. 17 as specific examples of favorable compounds among the compounds represented by the chemical formula (Host-B), which are suitably used for the organic EL device according to the present invention. However, the present invention is not limited to these Compounds.

Note that the above-mentioned having a carbazole structure can be synthesized in accordance with a method known per se (see, for example, Patent Literatures 4 and 5).

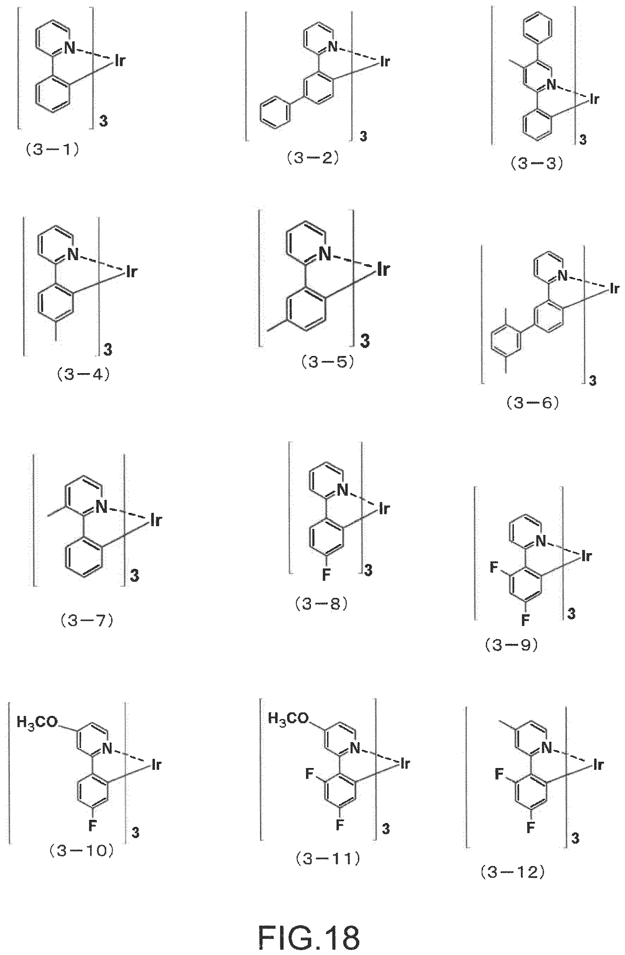

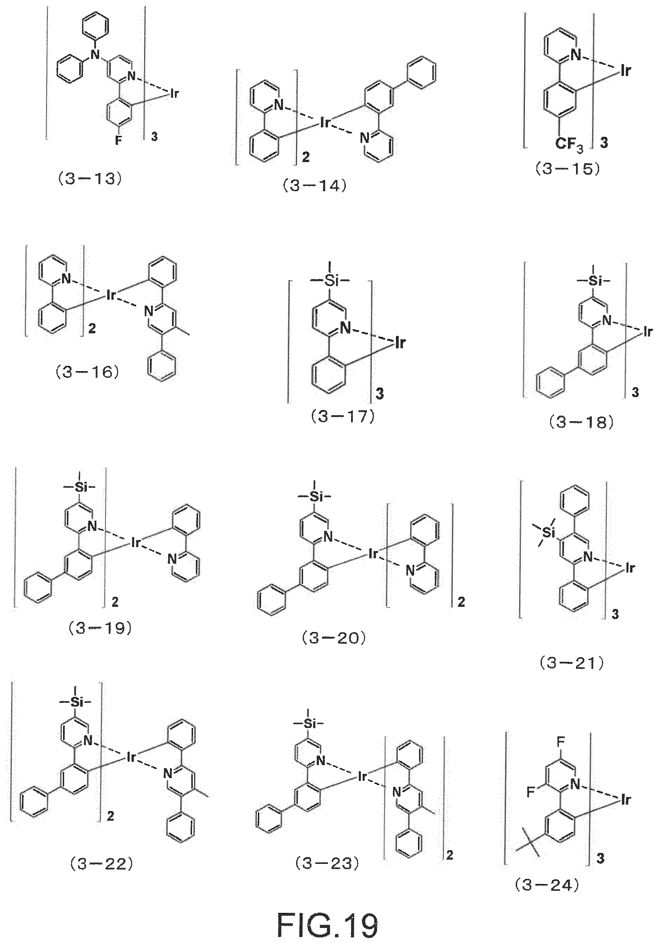

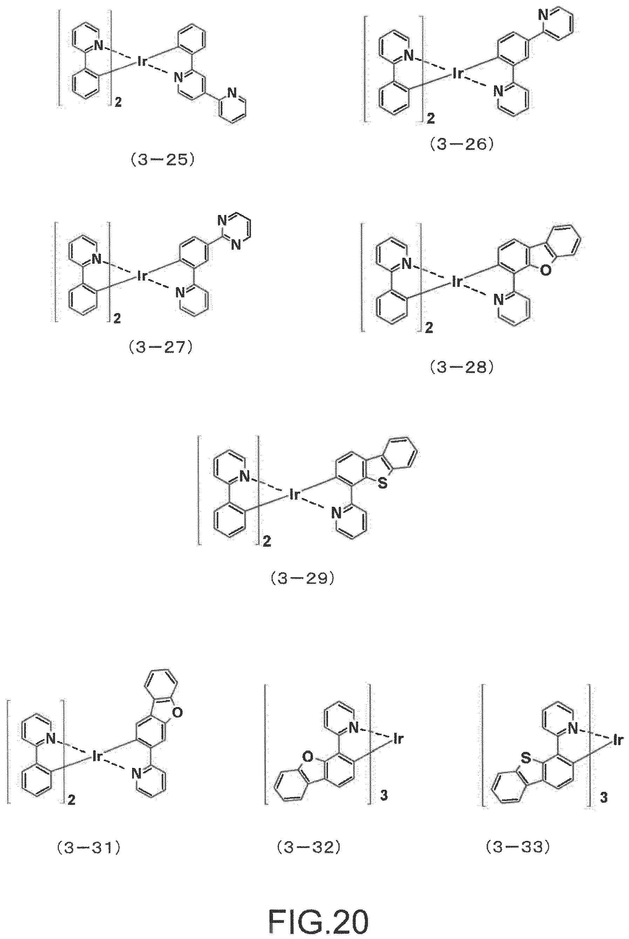

Compound (3-1) to Compound (3-33) are shown in Fig. 18 to Fig.20 as specific examples of favorable compounds among the compounds (metal complexes) represented by the chemical formula (3), which are suitably used for the organic EL device according to the present invention. However, the present invention is not limited to these Compounds.

Note that the above-mentioned iridium complex can be synthesized in accordance with a method known per se (see, for example, Patent Literatures 9 and 10).

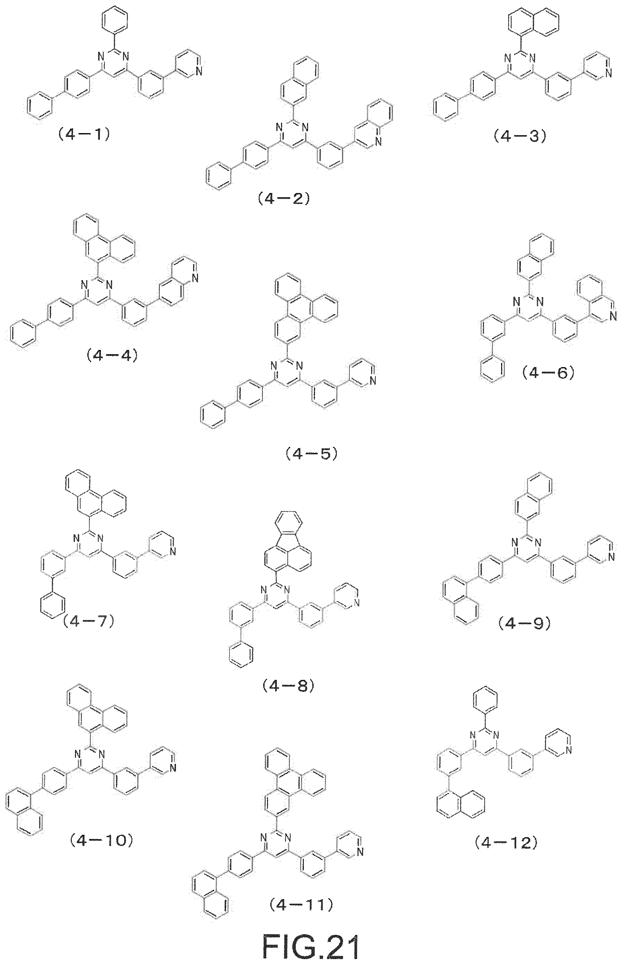

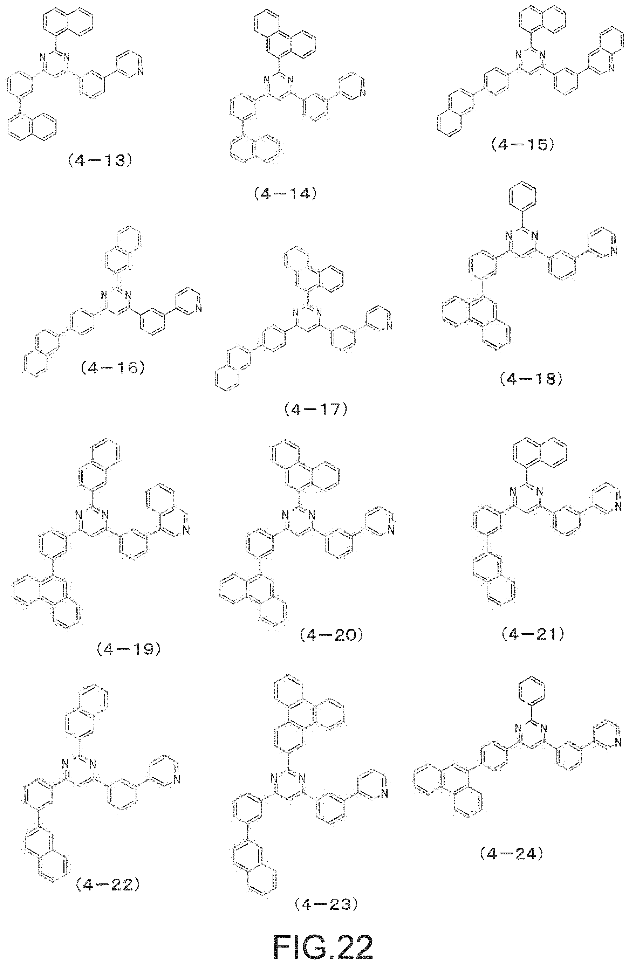

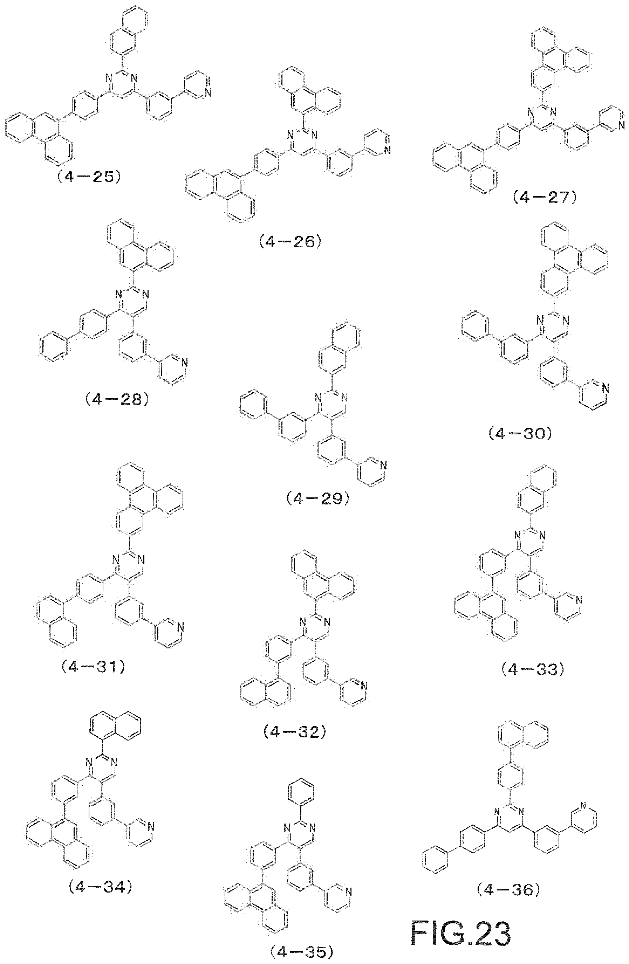

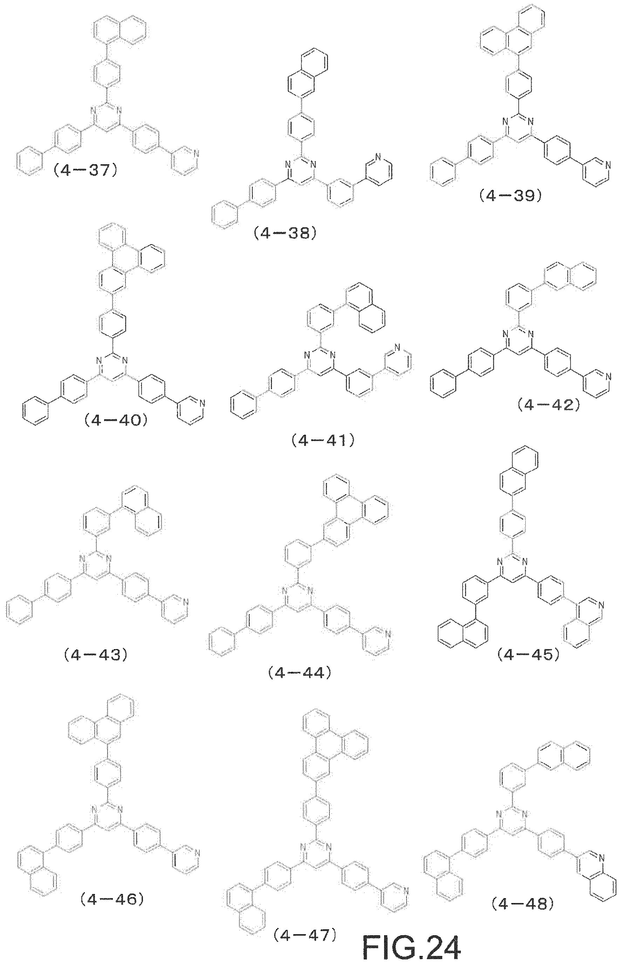

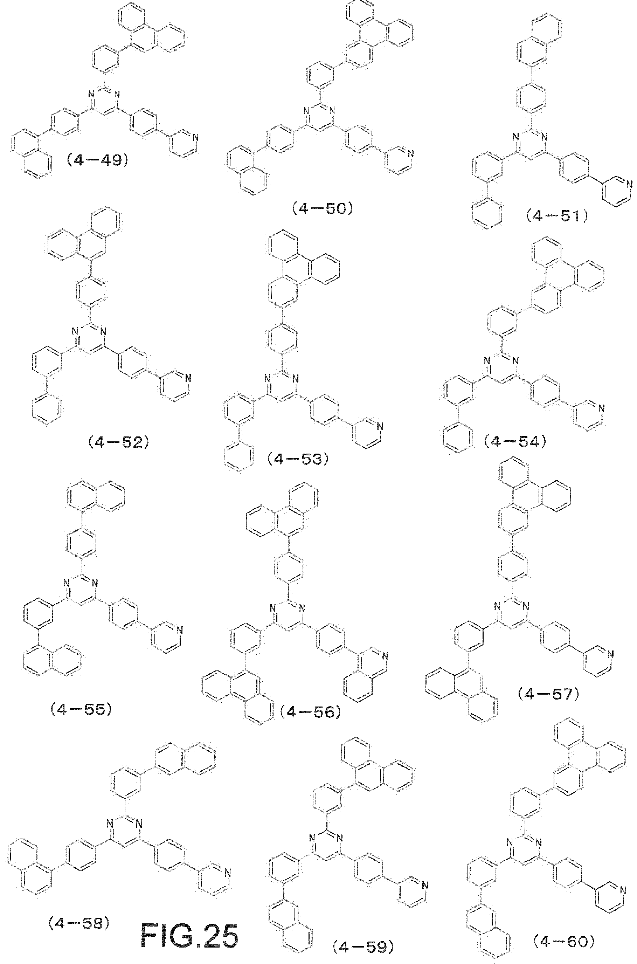

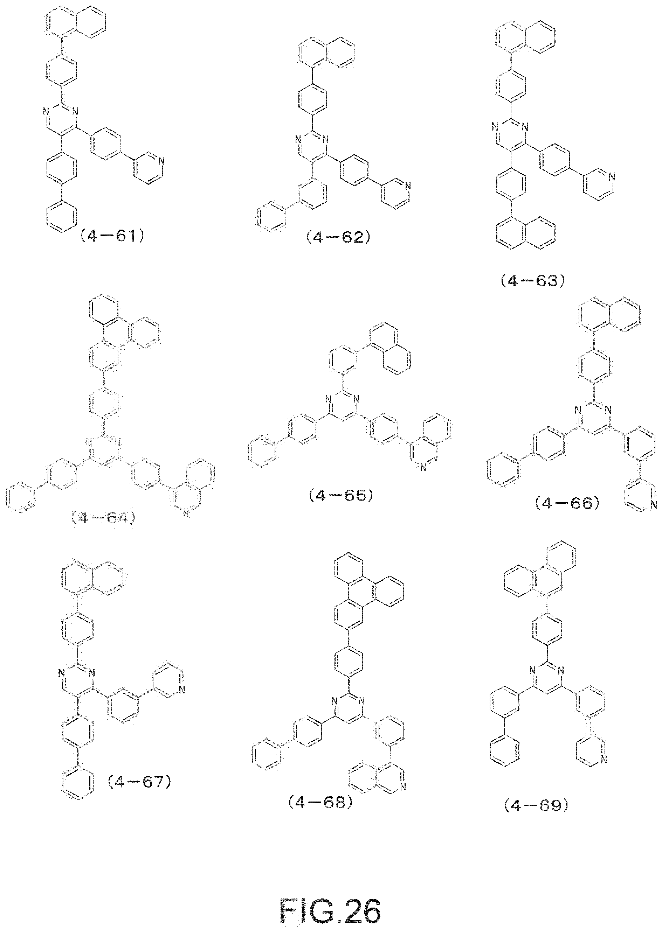

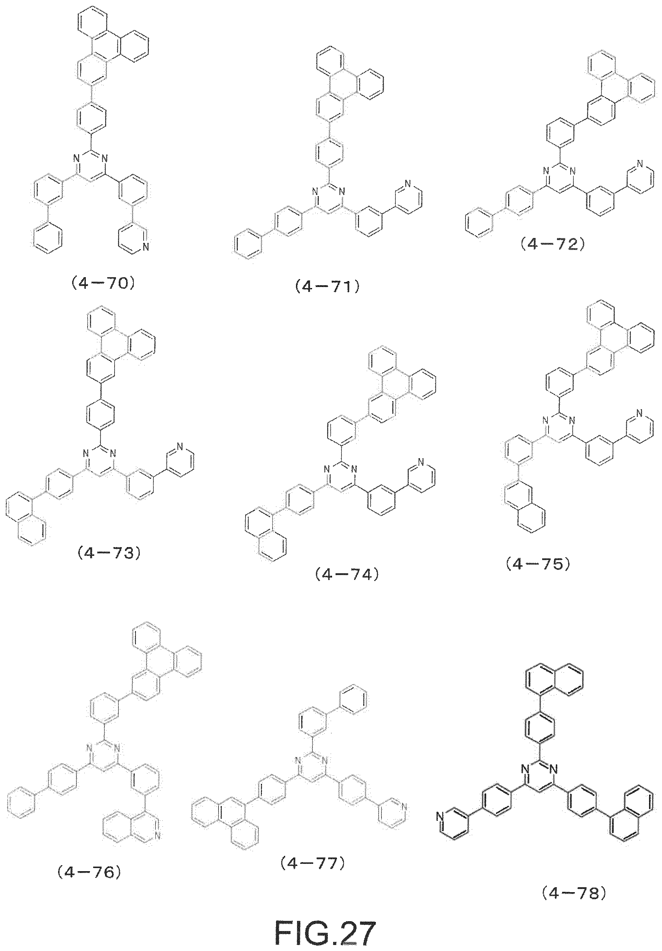

Compound (4-1) to Compound (4-78) are shown in Fig. 21 to Fig. 27 as specific examples of favorable compounds among the compounds having a pyrimidine structure, which are represented by the above-mentioned general formula (4) and suitably used for the organic EL device according to the present invention. However, the present invention is not limited to these Compounds.

Note that the above-mentioned compound having a pyrimidine structure can be synthesized by a method known per se (see, for example, Patent Literatures 10 and 11).

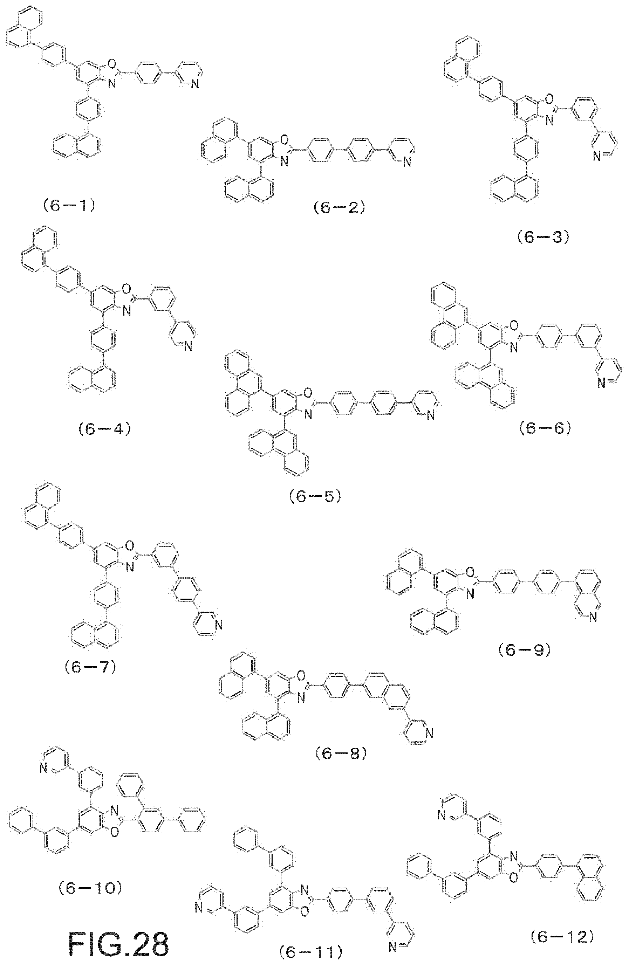

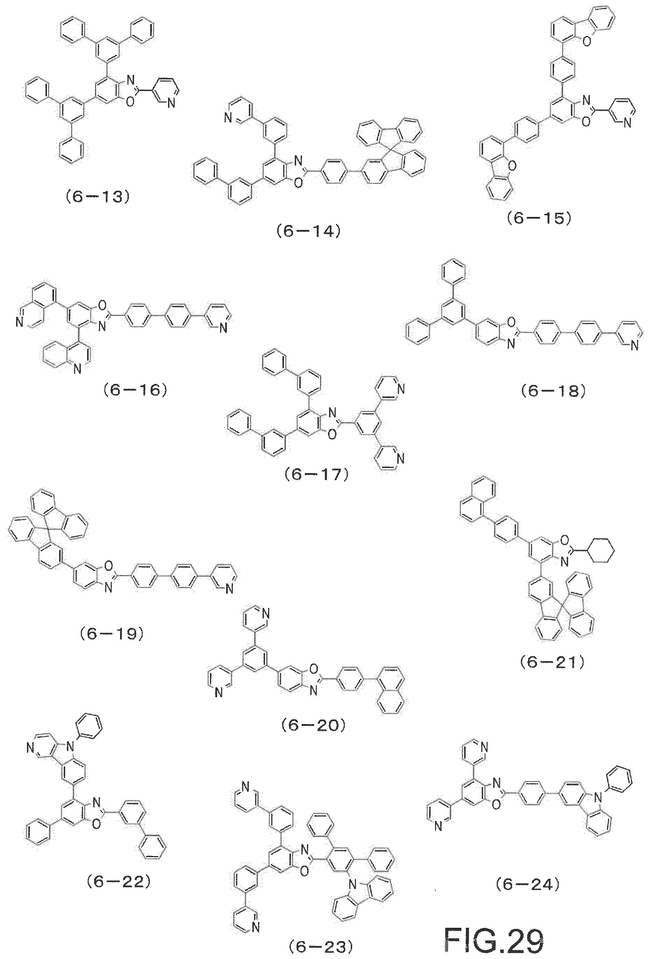

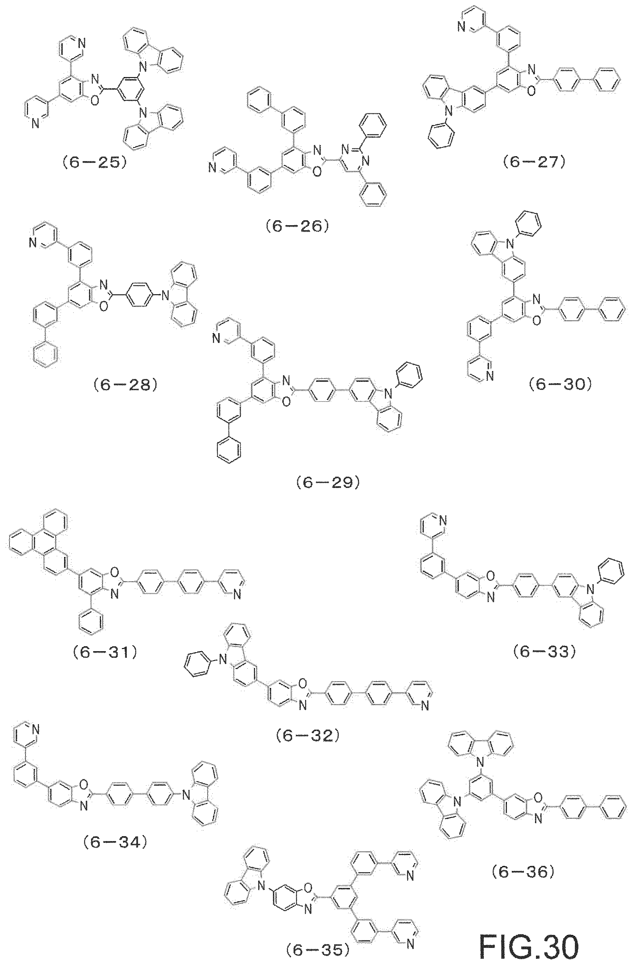

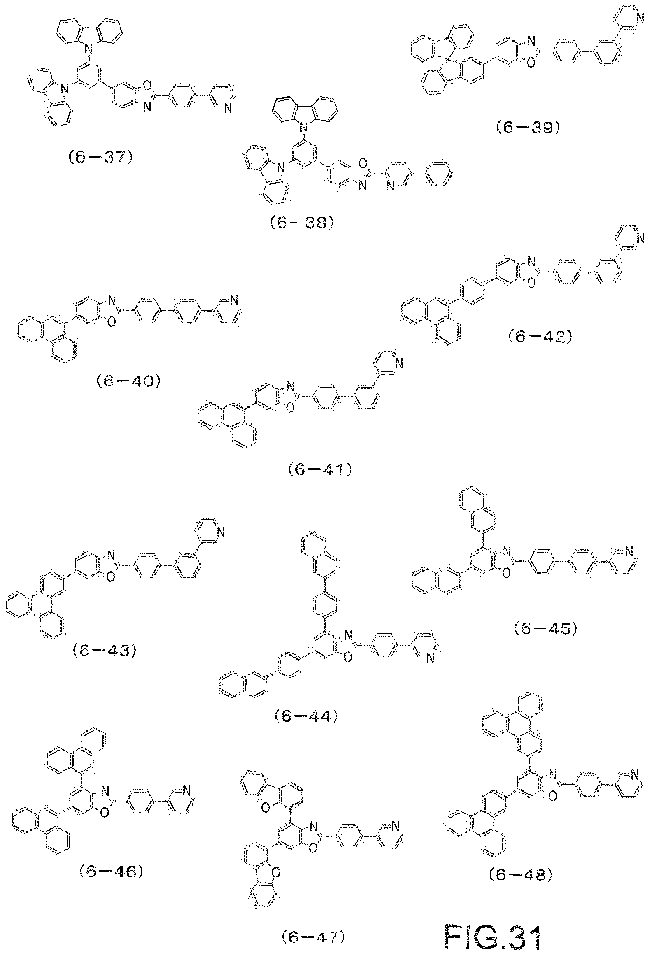

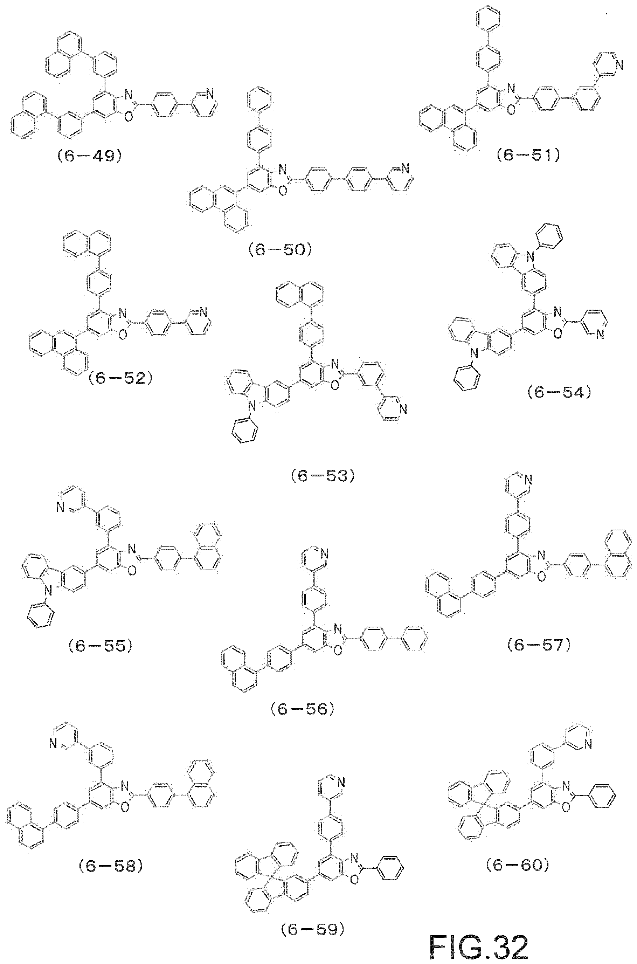

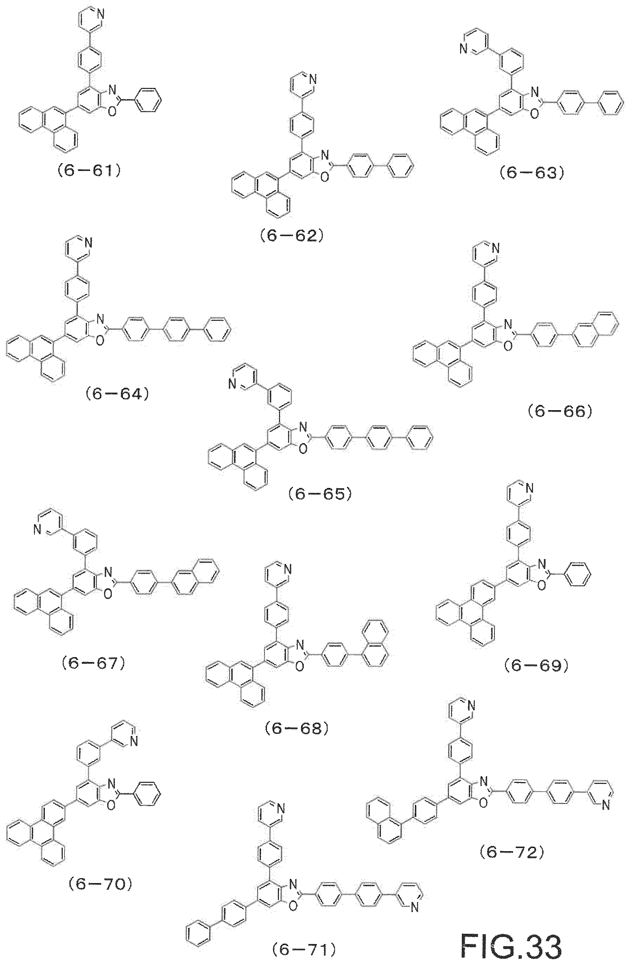

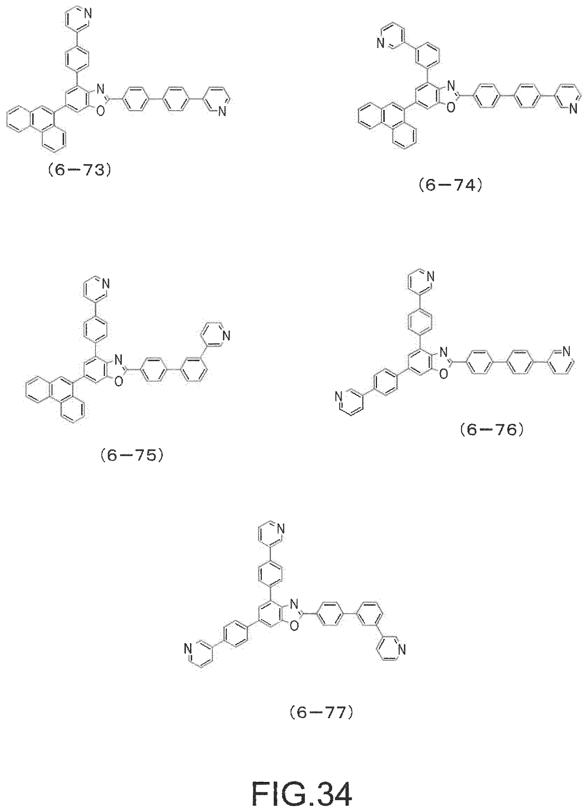

Compound (6-1) to Compound (6-77) are shown in Fig. 28 to Fig. 34 as specific examples of favorable compounds among the compounds having a benzoazole structure, which are represented by the above-mentioned general formula (6) and suitably used for the organic EL device according to the present invention. However, the present invention is not limited to these Compounds.

Note that the above-mentioned compound having a benzoazole structure can be synthesized in accordance with a method known per se (see, for example, Patent Literatures 13 and 14, and Non-Patent Literatures 3 and 4).

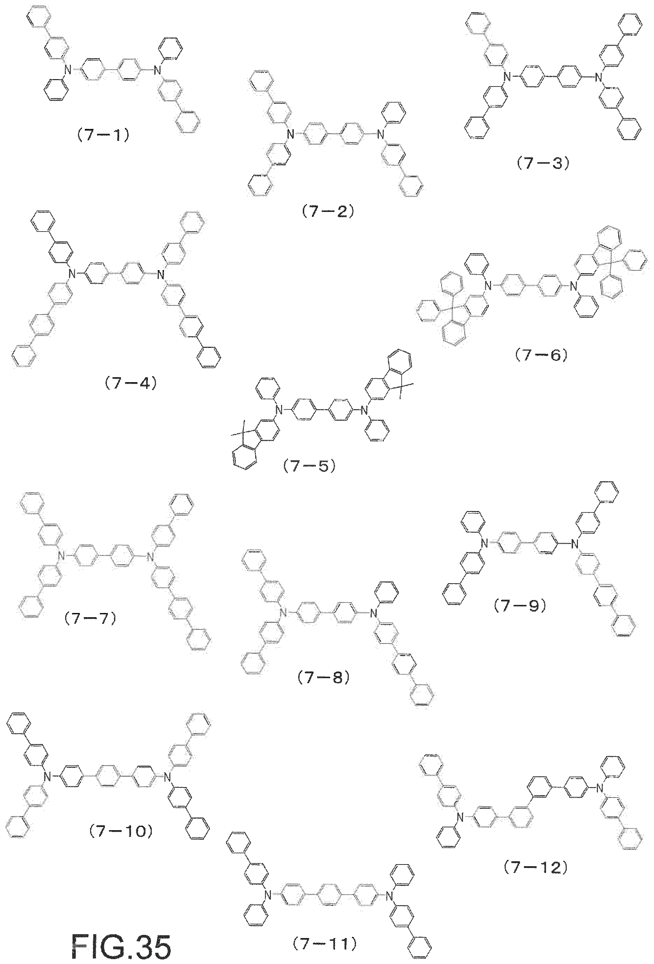

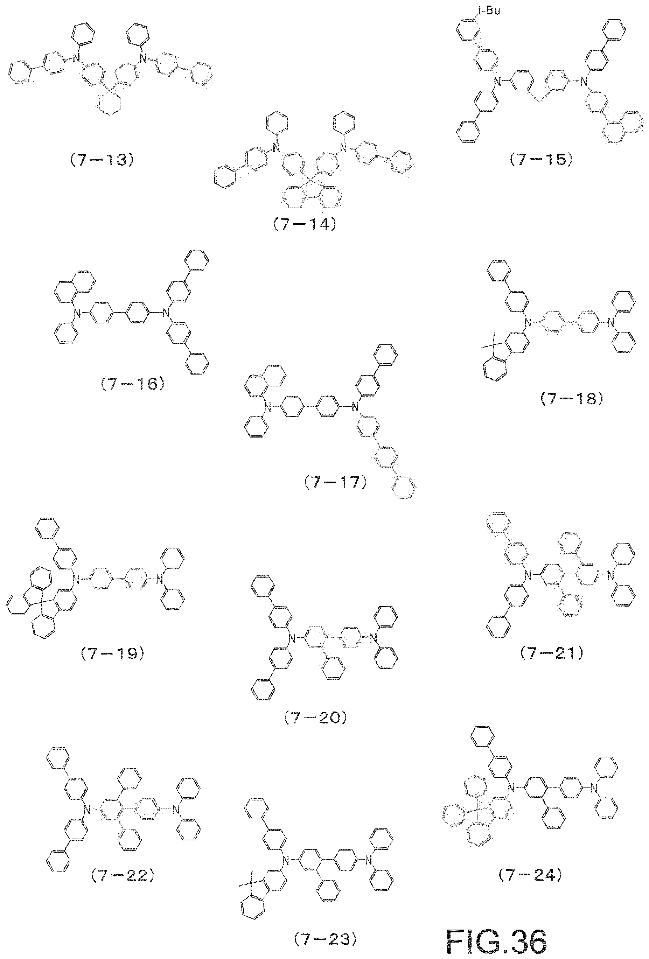

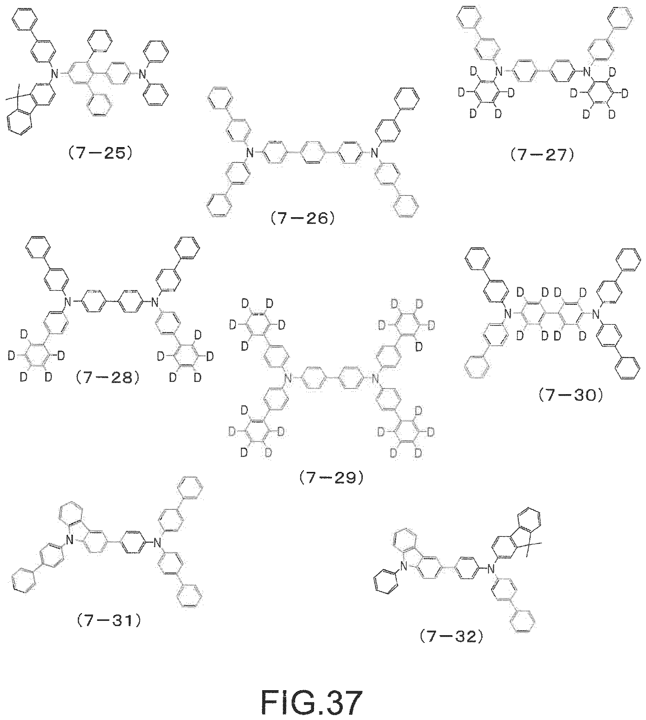

Compound (7-1) to Compound (7-32) are shown in fig. 35 to Fig. 37 as specific examples of favorable compounds among the triphenylamine derivatives, which are represented by the above-mentioned general formula (7) and suitably used for the organic EL device according to the present invention. However, the present invention is not limited to these Compounds.

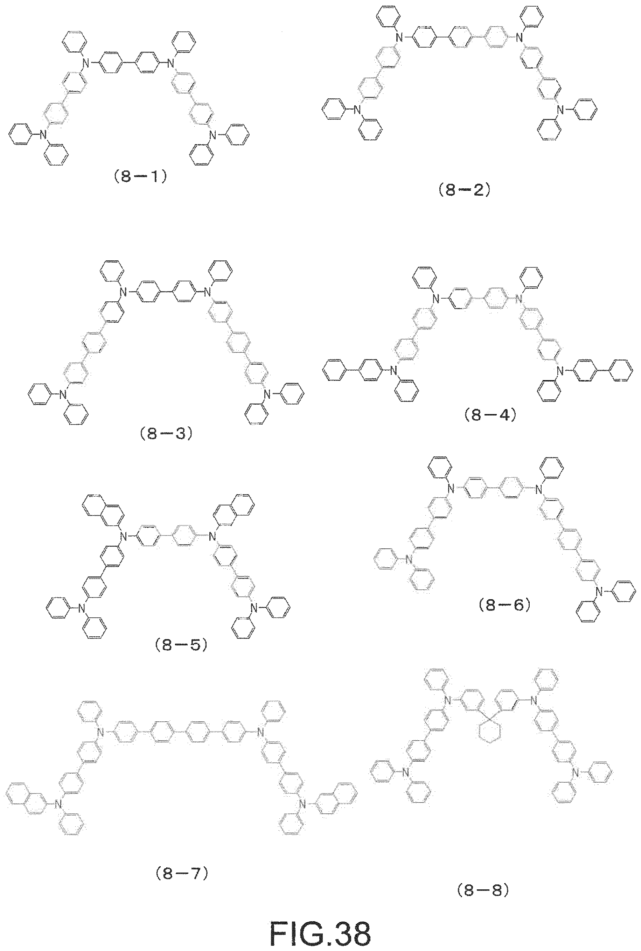

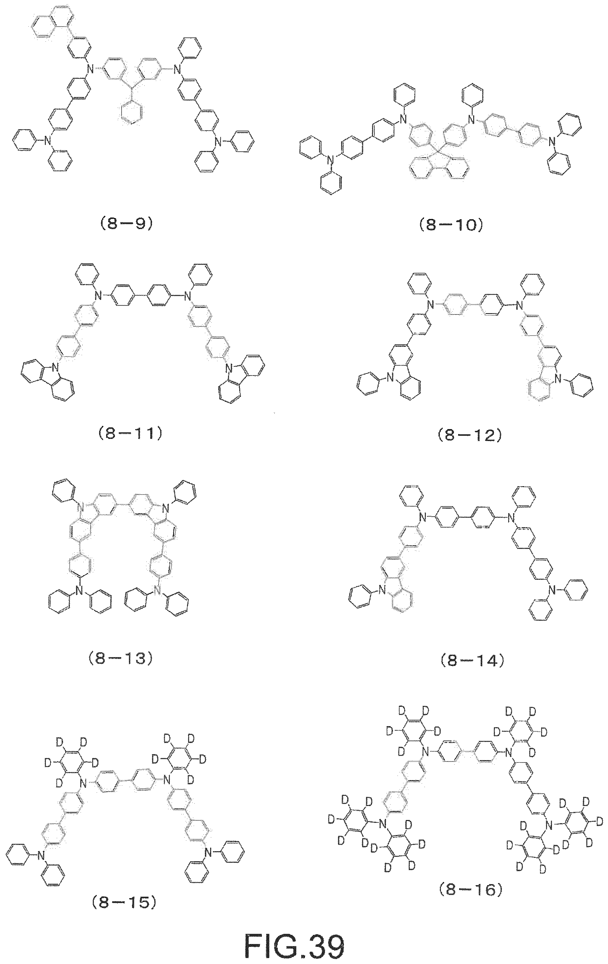

Compound (8-1) to Compound (8-16) are shown in Fig. 38 and Fig. 39 as specific examples of favorable compounds among the triphenylamine derivatives, which are represented by the above-mentioned general formula (8) and suitably used for the organic EL device according to the present invention. However, the present invention is not limited to these Compounds.

Note that the above-mentioned compound having a triarylamine structure can be synthesized in accordance with a method known per se (see, for example, Patent Literatures 1 and 2 and Patent Literature 15).

Purification for the general formulae (1) and (8), (HOST-A), and (HOST-B) was carried out by purification by column chromatography, adsorption purification with silica gel, activated carbon, activated clay, or the like, recrystallization with a solvent, a crystallization method, a sublimation purification method, or the like. Identification of the compounds was performed by NMR analysis. As physical property values, a melting point, a glass transition point (Tg), and a work function were measured. The melting point is an index of a vapor deposition property. The glass transition point (Tg) is an index of stability in a thin film state. The work function is an index of a hole transport property and a hole blocking property.

In addition, regarding the compound used for the organic EL device according to the present invention, those purified by purification by column chromatography, adsorption purification with silica gel, activated carbon, activated clay, or the like, recrystallization with a solvent, a crystallization method, or the like, and finally purified by a sublimation purification method were used.

The melting point and the glass transition point (Tg) were measured with a powder using a high sensitivity differential scanning calorimeter (DSC3100SA manufactured by Bruker AXS GmbH).

The work function was obtained by preparing a thin film of 100 nm on an ITO substrate and using an ionization potential measuring apparatus (PYS-202 manufactured by Sumitomo Heavy Industries, Ltd.).

Examples of the structure of the organic EL device according to the present invention include those including an anode, a hole injection layer, a first hole transport layer, a second hole transport layer, a light-emitting layer, an electron transport layer, an electron injection layer, and a cathode in the stated order on a substrate, and those including a hole blocking layer between the light-emitting layer and the electron transport layer. In the multilayer structures, several organic layers can be omitted or combined. For example, the electron injection layer and the electron transport layer may be combined. Further, two or more organic layers having the same function can be stacked. For example, two light-emitting layers may be stacked, or two electron transport layers may be stacked.

For the anode of the organic EL device according to the present invention, an electrode material having a large work function such as ITO and gold is used. As the hole injection layer of the organic EL device according to the present invention, a porphyrin compound typified by copper phthalocyanine, a starburst type triphenylamine derivative, an acceptor heterocyclic compound such as hexacyanoazatriphenylene, a coating type polymer material, or the like in addition to the arylamine compounds represented by the above-mentioned general formulae (7) and (8) can be used. These materials can be formed into a thin film by a known method such as a spin coat method and an ink jet method in addition to a vapor deposition method.

For the first hole transport layer of the organic EL device according to the present invention, the arylamine compounds represented by the above-mentioned general formulae (7) and (8) are more favorable. However, in addition thereto, a benzidine derivative such as N,N'-diphenyl-N,N'-di(m-tolyl)benzidine (hereinafter, abbreviated as TPD), N,N'-diphenyl-N,N'-di(α-naphthyl)benzidine (hereinafter, abbreviated as NPD), and N,N,N',N'-tetrabiphenylylbenzidine, 1,1-bis[4-(di-4-tolylamino)phenyl]cyclohexane (hereinafter, abbreviated as TAPC), or the like can be also used. These materials may be deposited alone. However, any of the materials may be mixed with another material and used as a single deposited layer. Further, a stacked structure of layers deposited alone, which are formed of any of the plurality of materials, layers mixed and deposited, which are formed of the plurality of materials, or at least one layer deposited alone, which is formed of any of the plurality of materials, and at least one layer mixed and deposited, which is formed of the plurality of materials, may be achieved. Further, for the hole injection/transport layer, a coating polymer material such as poly(3,4-ethylenedioxythiophene) (hereinafter, abbreviated as PEDOT)/poly(styrene sulfonate) (hereinafter, abbreviated as PSS) can be used. These materials can be formed into a thin film by a known method such as a spin coat method and an ink jet method in addition to a vapor deposition method.

Further, in the hole injection layer or the hole transport layer, those obtained by P-doping the material typically used for the respective layers with trisbromophenylaminehexachloroantimony or a radialene derivative(see, for example, Patent Literature 16), a polymer compound having, as a partial structure, the structure of a benzidine derivative such as TPD, or the like can be used.

For the second hole transport layer of the organic EL device according to the present invention, a compound having an electron blocking effect, such as a carbazole derivative such as 4,4',4''-tri(N-carbazolyl) triphenylamine(hereinafter, abbreviated as TCTA), 9,9-bis[4-(carbazol-9-yl)phenyl]fluorene, 1,3-bis(carbazol-9-yl)benzene (hereinafter, abbreviated as mCP), 2,2-bis(4-carbazol-9-ylphenyl)adamantane (hereinafter, abbreviated as Ad-Cz), and a compound having a triphenylsilyl group and a triarylamine structure typified by 9-[4-(carbazol-9-yl)phenyl]-9-[4-(triphenylsilyl)phenyl]-9H-fluorene in addition to the compound having an indenocarbazole ring structure according to the present invention, which is represented by the general formula (1), can be used. These materials may be deposited alone. However, any of the materials may be mixed with another material and used as a single deposited layer. Further, a stacked structure of layers deposited alone, layers mixed and deposited, or at least one layer deposited alone and at least one layer mixed and deposited may be achieved. These materials can be formed into a thin film by a known method such as a spin coat method and an ink jet method in addition to a vapor deposition method.

For the host of the light-emitting layer of the organic EL device according to the present invention, a host material having a hole transport property or a host material having an electron transport property can be used. As the host material having a hole transport property, a carbazole derivative such as 4,4'-di (N-carbazolyl) biphenyl (CBP), TCTA, and mCP in addition to the compound having a carbazole ring structure, which is represented by the above-mentioned general formula (HOST-B) and the compound having an indenocarbazole ring structure according to the present invention, which is represented by the general formula (1), can be used. As the host material having an electron transport property, p-bis(triphenylsilyl)benzene (UGH2), 2,2',2''-(1,3,5-phenylene)-tris(1-phenyl-1H-benzimidazole) (TPBi), or the like in addition to the compound having a nitrogen-containing heteroaromatic ring structure, which is represented by the above-mentioned general formula (HOST-A), can be used. These materials may be deposited alone. However, a plurality of materials may be mixed with each other and used as a single deposited layer. Further, a stacked structure of layers deposited alone, layers mixed and deposited, or at least one layer deposited alone and at least one layer mixed and deposited may be achieved. These materials can be formed into a thin film by a known method such as a spin coat method and an ink jet method in addition to a vapor deposition method.

In the present invention, it is favorable to use two or more compounds of a first host compound having electron transportability and a second host compound having hole transportability. One or two or more types of the above-mentioned second host compound may be used. The above-mentioned first host compound and the above-mentioned second host compound may have, for example, a weight ratio of 1:10 to 10:1.

As the above-mentioned first host compound of the light-emitting layer of the organic EL device according to the present invention, a compound having a nitrogen-containing heteroaromatic ring structure, which is represented by the above-mentioned general formula (HOST-A), is favorable. As the above-mentioned second host compound, a compound having a carbazole ring structure, which is represented by the above-mentioned general formula (HOST-B), or the compound having an indenocarbazole ring structure according to the present invention, which is represented by the general formula (1) is favorable.

In addition to the first host compound and the second host compound, one or more types of host compounds may be further contained.

As the phosphorescent light-emitting material of the organic EL device according to the present invention, the iridium complex represented by the general formula (3) of the present invention is favorable. However, in addition thereto, an organometallic compound containing Pt, Os, Ti, Zr, Hf, Eu, Tb, Tm, Fe, Co, Ni, Ru, Rh, Pd, or a combination thereof can be used. The dopant may be a red, green, or blue dopant, and an organic EL device having high performance can be prepared.

In order to avoid concentration quenching, it is favorable to dope the phosphorescent light-emitting material with the host material by co-deposition in the range of 1 to 30 weight percent with respect to the entire light-emitting layer.

These materials can be formed into a thin film by a known method such as a spin coat method and an ink jet method in addition to a vapor deposition method.

For the hole blocking layer of the organic EL device according to the present invention, the pyrimidine compound and the benzoazole compound represented by the above-mentioned general formulae (4) and (6) are more favorable. However, in addition thereto, a compound having a hole blocking effect, such as various rare earth complexes, an oxazole derivative, a triazole derivative, and a triazine derivative, in addition to a phenanthroline derivative such as bathocuproin (hereinafter, abbreviated as BCP) and a metal complex of a quinolinol derivative such as BAlq, can be used. These materials may double the material of the electron transport layer. These materials may be deposited alone. However, any of the materials may be mixed with another material and used as a single deposited layer. Further, a stacked structure of layers deposited alone, which are formed of any of the plurality of materials, layers mixed and deposited, which are formed of the plurality of materials, or at least one layer deposited alone, which is formed of any of the plurality of materials, and at least one layer mixed and deposited, which is formed of any of the plurality of materials, may be achieved. These materials can be formed into a thin film by a known method such as a spin coat method and an ink jet method in addition to a vapor deposition method.

For the electron transport layer of the organic EL device according to the present invention, the pyrimidine compound and the benzoazole compound represented by the above-mentioned general formulae (4) and (6) are more favorable. However, in addition thereto, various metal complexes, a triazole derivative, a triazine derivative, an oxadiazole derivative, a pyridine derivative, a benzimidazole derivative, a thiadiazole derivative, an anthracene derivative, a carbodiimide derivative, a quinoxaline derivative, a pyridoindole derivative, a phenanthroline derivative, a silole derivative, or the like in addition to a metal complex of a quinolinol derivative including Alq₃ and BAlq can be used. These materials may be deposited alone. However, any of the materials may be mixed with another material and used as a single deposited layer. Further, a stacked structure of layers deposited alone, which are formed of any of the plurality of materials, layers mixed and deposited, which are formed of the plurality of materials, or at least one layer deposited alone, which is formed of any of the plurality of materials, and at least one layer mixed and deposited, which is formed of any of the plurality of materials, may be achieved. These materials can be formed into a thin film by a known method such as a spin coat method and an ink jet method in addition to a vapor deposition method.

For the electron injection layer of the organic EL device according to the present invention, an alkali metal salt such as lithium fluoride and cesium fluoride, an alkaline earth metal salt such as magnesium fluoride, a metal complex of a quinolinol derivative such as lithium quinolinol, a metal oxide such as aluminum oxide, or the like can be used. However, this can be omitted in the favorable selection of the electron transport layer and the cathode.

Further, in the electron injection layer or the electron transport layer, those obtained by N-doping the organic compound typically used for the respective layers with a metal such as cesium, lithium fluoride, and ytterbium can be used.

In the cathode of the organic EL device according to the present invention, an electrode material having a low work function, such as aluminum and ytterbium, an alloy having a lower work function, such as a magnesium silver alloy, a magnesium indium alloy, and an aluminum magnesium alloy, or the like is used as the electrode material.

Hereinafter, the embodiment of the present invention will be specifically described by way of Examples. However, the present invention is not limited to the following Examples unless it exceeds the gist of thereof.

### (Example 1)

### <Synthesis of 12,12-dimethyl-10-phenyl-7-(9-phenyl-9H-carbazol-3-yl)-10,12-dihydroindeno[2,1-b]carbazole (Compound 1-1)>

N-(9,9-dimethyl-9H-fluorene-2-yl)-2-bromo-aniline: 18.5 g, potassium acetate: 6.98 g, and DMF: 95 ml were added to a reaction vessel purged with nitrogen, and nitrogen gas was bubbled for 1 hour. Tetrakis(triphenylphosphine)palladium: 1.18 g was added thereto, and the mixture was heated and stirred at 100°C for 11 hours. After the mixture was cooled to room temperature and the reaction solution was poured into 300 ml of water, extraction was performed with 300 ml of toluene. The obtained organic layer was repeatedly washed twice with 200 ml of water, dehydrated with anhydrous magnesium sulfate, and then concentrated under reduced pressure to obtain a crude product. The obtained crude product was purified by column chromatography (carrier: silica gel, eluent: toluene/n-hexane) to obtain a pale yellow powder 12,12-dimethyl-10,12-dihydroindeno[2,1-b]carbazole: 7.9 g (yield of 55.2%).

The obtained 12,12-dimethyl-10,12-dihydroindeno[2,1-b]carbazole: 7.8 g, iodobenzene: 3.7 ml, sodium bisulfite: 0.43 g, a copper powder: 0.17g, 3,5-di(tert-butyl)salicylic acid: 0.69 g, potassium carbonate: 5.71 g, and dodecylbenzene: 10 ml were added to a reaction vessel purged with nitrogen, and the mixture was heated and stirred at 170°C for 10 hours. After the mixture was cooled to 100°C,100 ml of toluene was added thereto, and extraction was performed, the mixture was concentrated under reduced pressure and crystalized with 30 ml of n-hexane to obtain a pale yellow powder of 12,12-dimethyl-10-phenyl-10,12-dihydroindeno[2,1-b]carbazole: 8.73 g (yield of 88.3%).

The obtained 12,12-dimethyl-10-phenyl-10,12-dihydroindeno[2,1-b]carbazole: 7.5 g and DMF: 53 ml were added to a reaction vessel. Under ice-cooling, N-bromosuccinimide: 3.72 g was added thereto, and the mixture was stirred for 9 hours and then left to stand overnight. Two hundred sixty ml of water was added thereto and filtration was performed to obtain a brownish white powder of 7-bromo-12,12-dimethyl-10-phenyl-10,12-dihydroindeno[2,1-b]carbazole: 8.67 g (yield of 94.6%).

The obtained 7-bromo-12,12-dimethyl-10-phenyl-10,12-dihydroindeno[2,1-b]carbazole: 2.0 g, 9-phenyl-3-(4,4,5,5-tetramethyl-1,3,2-dioxaborolan-2-yl)-9H-carbazole: 1.68 g, a mixed solvent of toluene/ethanol (4/1, v/v): 15 ml, and a 2M aqueous potassium carbonate solution: 3.4ml were added to a reaction vessel purged with nitrogen, and a nitrogen gas was bubbled for 30 minutes while irradiating ultrasonic waves. Tetrakis(triphenylphosphine)palladium: 0.26 g was added thereto, and the mixture was heated and stirred at 73°C for 5 hours. After the mixture was cooled to room temperature, toluene: 30 ml and water: 20 ml were added thereto, the mixture was separated, and an organic layer was collected. The organic layer was washed with saturated saline, dehydrated with anhydrous magnesium sulfate, and then concentrated under reduced pressure to obtain a crude product. The crude product was purified by column chromatography (carrier: silica gel, eluent: toluene/n-hexane) to obtain a white powder of 12,12-dimethyl-10-phenyl-7-(9-phenyl-9H-carbazol-3-yl)-10,12-dihydroindeno[2,1-b]carbazole: 1.5 g (yield of 54.7%).

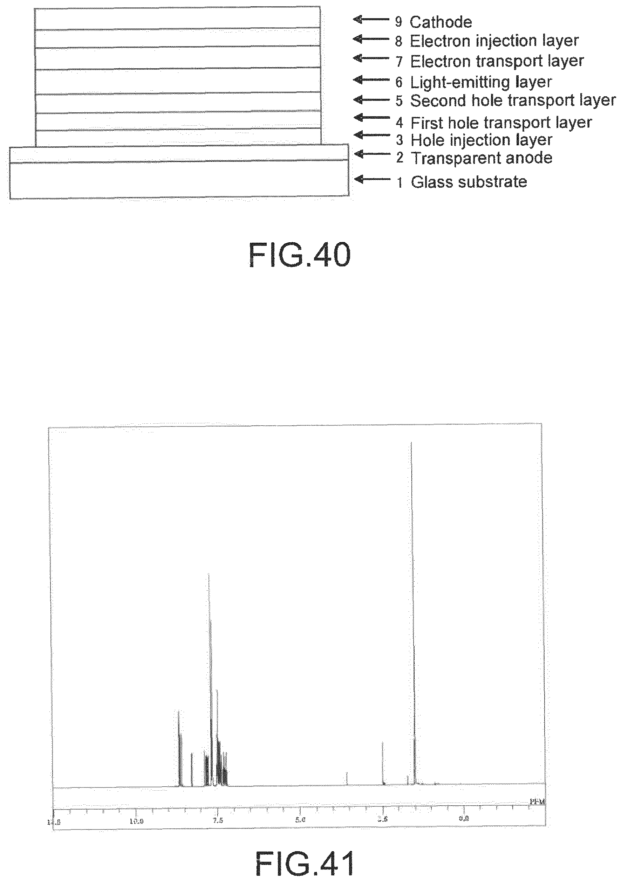

The structure of the obtained white powder was identified using NMR. The ¹H-NMR measurement results are shown in Fig. 41.

The following 32 hydrogen signals were detected by ¹H-NMR(THF-d₈).

δ(ppm)=8.66(1H), 8.64(1H), 8.59(1H), 8.23-8.29(1H), 7.88-7.90(1H), 7.83-7.85(1H), 7.78-7.80(1H), 7.66-7.71(8H), 7.42-7.53(7H), 7.37-7.40(1H), 7.31-7.33(1H), 7.26-7.29(1H), 7.21-7.24(1H), 1.51(6H).

### (Example 2)

### <Synthesis of 12,12-dimethyl-10-phenyl-7-(4-diphenylamino-phenyl)-10,12-dihydroindeno[2,1-b]carbazole(Compound 1-2)>

The 7-bromo-12,12-dimethyl-10-phenyl-10,12-dihydroindeno[2,1-b]carbazole: 2.0 g synthesized in Example 1, 4-diphenylamino-phenyl boronic acid: 1.32 g, a mixed solvent of toluene/ethanol(4/1, v/v): 15 ml, and a 2M aqueous potassium carbonate solution: 3.4 ml were added to a reaction vessel purged with nitrogen, and a nitrogen gas was bubbled for 30 minutes while irradiating ultrasonic waves. Tetrakis(triphenylphosphine)palladium: 0.26 g was added thereto, and the mixture was heated and stirred at 73°C for 5 hours. After the mixture was cooled to room temperature, toluene: 30 ml and water: 20 ml were added thereto, the mixture was separated, and an organic layer was collected. The organic layer was washed with saturated saline, dehydrated with anhydrous magnesium sulfate, and then concentrated under reduced pressure to obtain a crude product. The crude product was purified by column chromatography (carrier: silica gel, eluent: toluene/n-hexane) to obtain a pale yellowish white powder of 12,12-dimethyl-10-phenyl-7-(4-diphenylamino-phenyl)-10,12-dihydroindeno[2,1-b]carbazole; 1.6 g (yield of 58.4%).

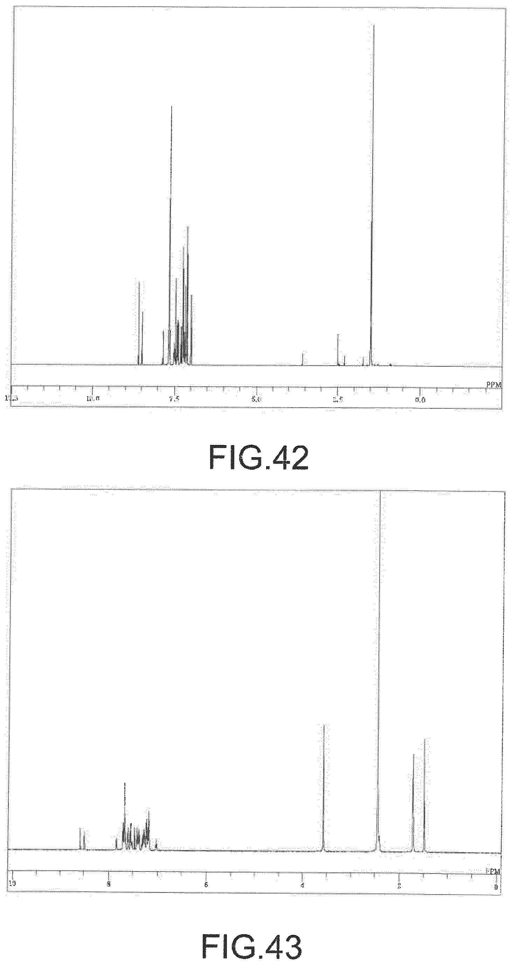

The structure of the obtained white powder was identified using NMR. The ¹H-NMR measurement results are shown in Fig. 42.

The following 34 hydrogen signals were detected by ¹H-NMR(THF-d₈).

δ(ppm)=8.60(1H), 8.50(1H), 7.85-7.86(1H), 7.64-7.69(7H), 7.48-7.52(2H), 7.40-7.43(2H), 7.30-7.32(1H), 7.24-7.26(4H), 7.21-7.22(1H), 7.17-7.18(2H), 7.11-7.13(4H), 6.98-7.01(2H), 1.49(6H).

### (Example 3)

### <Synthesis of 7-[4-{(biphenyl-4-yl)-phenylamino}-phenyl]-12,12-dimethyl-10-phenyl-10,12-dihydroindeno[2,1-b]carbazole (Compound-3)>

The 7-bromo-12,12-dimethyl-10-phenyl-10,12-dihydro-indeno[2,1-b]carbazole synthesized in Example 1: 3.0g, (biphenyl-4-yl)-[4-(4,4,5,5-tetramethyl-[1,3,2]dioxaboran-2-yl)phenyl]-phenylamine: 3.7 g, a mixed solvent of toluene/ethanol (4/1, v/v): 50 ml, and a 2M aqueous potassium carbonate solution: 10 ml were added to a reaction vessel purged with nitrogen, and a nitrogen gas was bubbled for 30 minutes while irradiating ultrasonic waves. Tetrakis(triphenylphosphine)palladium: 0.4 g was added thereto, and the mixture was heated and stirred at 73°C for 8 hours. The mixture was cooled to room temperature, and the precipitated crude product was collected by filtration. After adding 1,2-dichlorobenzene: 140 ml to the crude product and dissolving them while heating, insoluble materials were removed by filtration and then the filtrate was concentrated under reduced pressure. The obtained product was purified by recrystallization using 1,2-dichlorobenzene: 100 ml to obtain a white powder 7-[4-{(biphenyl-4-yl)-phenylamino}-phenyl]-12,12-dimethyl-10-phenyl-10,12-dihydroindeno[2,1-b]carbazole: 2.7g(yield of 57.8%).

The structure of the obtained white powder was identified using NMR. The ¹H-NMR measurement results are shown in Fig. 43.

The following 38 hydrogen signals were detected by ¹H-NMR (THF-d₈).

δ(ppm)=8.60(1H), 8.50(1H), 7.85(1H), 7.72-7.65(7H), 7.61(2H), 7.55(2H), 7.52(1H), 7.47(1H), 7.43-7.37(4H), 7.31-7.16(11H), 7.03(1H), 1.49(6H).

### (Example 4)

### <Synthesis of 7-[4-{bis(biphenyl-4-yl)amino}-phenyl]-12,12-dimethyl-10-phenyl-10,12-dihydroindeno[2,1-b]carbazole (Compound-4)>

The 7-bromo-12,12-dimethyl-10-phenyl-10,12-dihydro-indeno[2,1-b]carbazole synthesized in Examples 1: 3.0 g, bis(biphenyl-4-yl)-[4-(4,4,5,5-tetramethyl-[1,3,2]dioxaboran-2-yl)phenyl]amine: 4.3 g, a mixed solvent of toluene/ethanol (4/1, v/v): 50 ml, and a 2M aqueous potassium carbonate solution: 10 ml were added to a reaction vessel purged with nitrogen, and a nitrogen gas was bubbled for 30 minutes while irradiating ultrasonic waves. Tetrakis(triphenylphosphine)palladium: 0.4 g was added thereto, and the mixture was heated and stirred at 73°C for 8 hours. The mixture was cooled to room temperature, and the precipitated crude product was collected by filtration. After adding 1,2-dichlorobenzene: 140 ml to the crude product and dissolving them while heating, insoluble materials were removed by filtration and then, the filtrate was concentrated under reduced pressure. The obtained product was purified by recrystallization using 1,2-dichlorobenzene: 100 ml to obtain a white powder of 7-[4-{bis(biphenyl-4-yl)amino}-phenyl]-12,12-dimethyl-10-phenyl-10,12-dihydroindeno[2,1-b]carbazole: 3.7 g (yield of 71.6%).

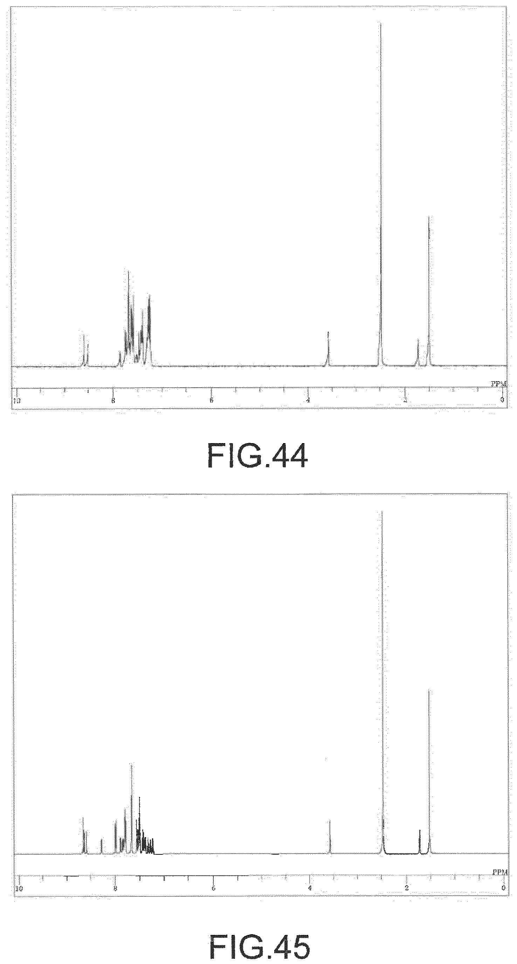

The structure of the obtained white powder was identified using NMR. The ¹H-NMR measurement results are shown in Fig. 44.

The following 42 hydrogen signals were detected by ¹H-NMR(THF-d₈).

δ(ppm)=8.60(1H), 8.52(1H), 7.85(1H), 7.75-7.57(15H), 7.53(1H), 7.47(1H), 7.43-7.38(6H), 7.32-7.22(10H), 1.49(6H).

### (Example 5)

### <Synthesis of 10-(biphenyl-4-yl)-12,12-dimethyl-7-(9-phenyl-9H-carbazol-3-yl)-10,12-dihydroindeno[2,1-b]carbazole (Compound-5)>

The 12,12-dimethyl-10,12-dihydroindeno[2,1-b]carbazole synthesized in Example 1: 35.5 g, 4-bromobiphenyl: 35.0 g, sodium bisulfite: 6.0 g, a copper powder: 2.4 g, 3,5-di(tert-butyl)salicylic acid: 9.4 g, potassium carbonate: 31.2 g, and dodecylbenzene: 52 ml were added to a reaction vessel purged with nitrogen, and the mixture was heated and stirred at 190°C for 26 hours. The mixture was cooled to 120°C, toluene: 35 ml was added thereto, the mixture was stirred, and a crude product was collected by filtration. After adding toluene 1.6 L to the crude product, heating them, and performing extraction at 110°C, the obtained product was cooled to room temperature and was concentrated under reduced pressure. The obtained product was crystalized with methanol: 120 ml to obtain a white powder of 10-(biphenyl-4-yl)-12,12-dimethyl-10,12-dihydroindeno[2,1-b]carbazole: 48.5 g (yield of 88.1%).

After adding the obtained 10-(biphenyl-4-yl)-12,12-dimethyl-10,12-dihydroindeno[2,1-b]carbazole: 42.5 g and DMF: 2.5 L to a reaction vessel and dissolving the mixture by heating it to 70°C, the mixture was cooled to room temperature, N-bromosuccinimide: 17.4 g was added thereto, and the mixture was stirred for 7 hours. Water: 2.5 L was added thereto, and filtration was performed to obtain a white powder of 10-(biphenyl-4-yl)-7-bromo-12,12-dimethyl-10,12-dihydroindeno[2,1-b]carbazole: 34.9g(yield of 69.5%).

The obtained 10-(biphenyl-4-yl)-7-bromo-12, 12-dimethyl-10,12-dihydroindeno[2,1-b]carbazole: 16.5 g, 9-phenyl-3-(4,4,5,5-tetramethyl-1,3,2-dioxaborolan-2-yl)-9H-carbazole: 14.2 g, a mixed solvent of toluene/ethanol (4/1, v/v): 250 ml, and a 2M aqueous potassium carbonate solution: 48 ml were added to a reaction vessel purged with nitrogen, and a nitrogen gas was bubbled for 30 minutes while irradiating ultrasonic waves. Tetrakis(triphenylphosphine)palladium: 1.9 g was added thereto, and the mixture was heated and stirred at 73°C for 5 hours. The mixture was cooled to room temperature, and the precipitated crude product was collected by filtration. After adding 1,2-dichlorobenzene: 450 ml to the crude product and dissolving the mixture while heating, insoluble materials were removed by filtration and then, the filtrate was concentrated under reduced pressure. The obtained product was purified by crystallization with 1,2-dichlorobenzene: 150 ml and n-hexane: 300 ml to obtain a white powder of 10-(biphenyl-4-yl)-12,12-dimethyl-7-(9-phenyl-9H-carbazol-3-yl)-10,12-dihydroindeno[2,1-b]carbazole: 9.8 g (yield of 45.2%).

The structure of the obtained white powder was identified using NMR. The ¹H-NMR measurement results are shown in Fig. 45.

The following 36 hydrogen signals were detected by ¹H-NMR (THF-d₈).

δ(ppm)=8.69(1H), 8.64(1H), 8.59(1H), 8.28(1H), 7.99(2H), 7.89(1H), 7.85-7.78(6H), 7.66(4H), 7.56-7.49(6H), 7.44-7.37(4H), 7.32(1H), 7.27(1H), 7.23(1H), 1.52(6H).

### (Example 6)

### <Synthesis of 10-(biphenyl-4-yl)-7-[4-bis(biphenyl-4-yl)amino-phenyl]-12,12-dimethyl-10,12-dihydroindeno[2,1-b]carbazole (Compound-6)>

The 10-(biphenyl-4-yl)-7-bromo-12,12-dimethyl-10,12-dihydroindeno[2,1-b]carbazole synthesized in Example 6: 13.0 g, bis(biphenyl-4-yl)-[4-(4,4,5,5-tetramethyl-[1,3,2]dioxaboran-2-yl)phenyl]amine: 15.9 g, a mixed solvent of toluene/ethanol (4/1, v/v): 250 ml, and a 2M aqueous potassium carbonate solution: 51 ml were added to a reaction vessel purged with nitrogen, and a nitrogen gas was bubbled for 30 minutes while irradiating ultrasonic waves. Tetrakis(triphenylphosphine)palladium: 2.1 g was added thereto, and the mixture was heated and stirred at 73°C for 10 hours. The mixture was cooled to room temperature, and the precipitated crude product was collected by filtration. After adding 1,2-dichlorobenzene: 1.7L to the crude product and dissolving them while heating, insoluble materials were removed by filtration and then cooled to room temperature. The precipitated solid was collected by filtration and the obtained product was purified by recrystallization using 1,2-dichlorobenzene: 1.7 L to obtain a white powder of 10-(biphenyl-4-yl)-7-[4-bis(biphenyl-4-yl)amino-phenyl]-12,12-dimethyl-10,12-dihydroindeno[2,1-b]carbazole: 13.4 g (yield of 63.8%).

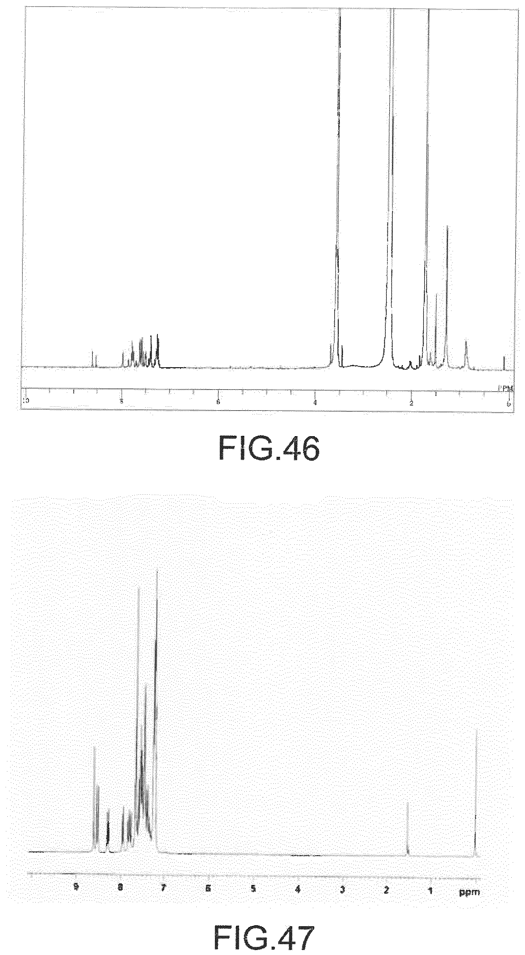

The structure of the obtained white powder was identified using NMR. The ¹H-NMR measurement results are shown in Fig. 46.

The following 46 hydrogen signals were detected by ¹H-NMR(THF-d₈).

δ(ppm)=8.62(1H), 8.54(1H), 7.98(2H), 7.86(1H), 7.78(4H), 7.75(2H), 7.70(1H), 7.63(4H), 7.58(4H), 7.55(1H), 7.50(3H), 7.43(1H), 7.40(4H), 7.33-7.21(11H), 1.51(6H).

### (Example 7)

### <Synthesis of 5,7-dihydro-5,7,7-triphenyl-2-(9-phenyl-9H-carbazol-3-yl) indeno[2,1-b] carbazole (Compound-20)>

5,7-dihydro-5,7-diphenylindeno[2,1-b]carbazole: 31.3 g, iodobenzene: 23.5 g, potassium carbonate: 15.9 g, sodium hydrogen nitrite: 1.2 g, 3,5-di-t-butylsalicylic acid: 1.9 g, a copper powder: 0.5g, and dodecylbenzene: 31 mL were added to a reaction vessel purged with nitrogen, and the mixture was heated and stirred at 190°C for 17 hours. The mixture was diluted with toluene, and insoluble materials were removed by filtration. The filtrate was concentrated, and the solid precipitated by adding heptane was collected by filtration to obtain a yellow powder of 5,7-dihydro-5,7,7-triphenylindeno[2,1-b]carbazole: 35.3 g (yield of 94%) .

The obtained 5,7-dihydro-5,7,7-triphenylindeno[2,1-b]carbazole: 35.0 g and dichloromethane: 350 mL was added to a reaction vessel purged with nitrogen, and the mixture was cooled in an ice bath. N-bromosuccinimide: 12.9 g was slowly added thereto, and the mixture was heated to 40°C and stirred for 24 hours. The solid precipitated by adding methanol is collected by filtration, and the obtained solid was washed with methanol to obtain a white powder of 2-bromo-5,7-dihydro-5,7,7-triphenylindeno[2,1-b]carbazole: 39.5 g (yield of 97%).

The obtained 2-bromo-5,7-dihydro-5,7,7-triphenylindeno[2,1-b]carbazole: 39.5 g, toluene: 320 mL, ethanol: 50 mL, 9-phenylcarbazole-3-a boronic acid: 24.2 g, and then an aqueous solution obtained by dissolving potassium carbonate: 14.6 g in water: 52 mL in advance were added to a reaction vessel purged with nitrogen, and a nitrogen gas was bubbled for 30 minutes while irradiating ultrasonic waves. Tetrakistriphenylphosphine palladium: 1.6 g was added thereto, and the mixture was heated and stirred at 72°C for 18 hours. The mixture was cooled to room temperature, and an organic layer was collected by a liquid separation operation. After sequentially washing the organic layer using water and then saturated saline, the obtained product was dried using anhydrous magnesium sulfate and concentrated to obtain a crude product. The crude product was dissolved in toluene: 360 mL, and adsorption purification using silica gel was performed, followed by adsorption purification using activated clay. The filtrate was concentrated, and the solid precipitated by adding acetone was collected by filtration. The obtained solid was recrystallized with toluene and acetone to obtain a white powder of 5,7-dihydro-5,7,7-triphenyl-2-(9-phenyl-9H-carbazol-3-yl)indeno[2,1-b]carbazole(Compound-20): 32.3 g (yield of 64%).

The structure of the obtained white powder was identified using NMR. The ¹H-NMR measurement results are shown in Fig. 47.

The following 36 hydrogen signals were detected by ¹H-NMR(CDCl₃).

δ(ppm)=7.63(1H), 8.56(2H), 8.30(1H), 7.80-7.95(3H), 7.22-7.77(29H).

### (Example 8)

The melting point and the glass transition point of the indenocarbazole compound represented by the general formula (1) were measured using a high sensitivity differential scanning calorimeter (DSC3100SA manufactured by Bruker AXS GmbH). Glass transition point Compound of Example 1 of present invention 148°C Compound of Example 2 of present invention 132°C Compound of Example 3 of present invention 143°C Compound of Example 4 of present invention 162°C Compound of Example 5 of present invention 163°C Compound of Example 6 of present invention 170°C Compound of Example 7 of present invention 175°C

The compound of the present invention has a glass transition point of 100°C or higher, which indicates that the thin film state is stable in the compound of the present invention.

### (Example 9)

A deposition film of 100 nm was prepared on an ITO substrate by using the indenocarbazole compound represented by the general formula (1), and the work function thereof was measured by an ionization potential measuring apparatus (PYS-202 manufactured by Sumitomo Heavy Industries, Ltd.). Work function Compound of Example 1 of present invention 5.79 eV Compound of Example 2 of present invention 5.60 eV Compound of Example 3 of present invention 5.65 eV Compound of Example 4 of present invention 5.64eV Compound of Example 5 of present invention 5.76eV Compound of Example 6 of present invention 5.79eV Compound of Example 7 of present invention 5.77eV

It can be seen that the indenocarbazole compound represented by the general formula (1) has favorable hole transport performance because it has a more favorable energy level than the work function that a general hole transport material such as NPD and TPD has, which is 5.4 eV.

### (Example 10)

The organic EL device was prepared by depositing a hole injection layer 3, a hole transport layer 4, a second hole transport layer 5, a light-emitting layer 6, an electron transport layer 7, an electron injection layer 8, and a cathode (aluminum electrode) 9 in the stated order on a transparent anode 2, which has been formed on a glass substrate 1 as an ITO electrode in advance, as shown in Fig. 40.

Specifically, after performing, in isopropyl alcohol for 20 minutes, ultrasonic cleaning on the glass substrate 1 on which ITO having a film thickness of 150 nm was formed, the glass substrate 1 was dried for 10 minutes on a hot plate heated to 200°C. After that, UV ozone treatment was performed for 15 minutes, and then, the ITO-attached glass substrate was mounted in a vacuum deposition machine. The pressure in the vacuum deposition machine was reduced to 0.001 Pa or less. Subsequently, a film of a compound (Acceptor-1) having the following structural formula and the Compound (7-1) was formed, as the hole injection layer 3, to have a film thickness of 10 nm and cover the transparent anode 2 by binary deposition at a deposition rate in which the ratio of the deposition rates of (Acceptor-1) and the Compound (7-1) was 3:97. As the first hole transport layer4, a film of the Compound (7-1) was formed on the hole injection layer 3 to have a film thickness of 70 nm. As the second hole transport layer 5, a film of the Compound (1-20) according to Example 7 was formed on the first hole transport layer 4 to have a film thickness of 10 nm. The light-emitting layer 5 was formed on the second hole transport layer 5 by simultaneously using the above-mentioned first host compound(A-19) and the above-mentioned second host compound(B-22) as hosts and doping the iridium compound (3-3) to 5 wt% as a dopant to have a film thickness of 40 nm by vacuum deposition. Here, the above-mentioned first host compound (A-20) and the above-mentioned second host compound (B-10) were used in the ratio of 1:1.

Next, a film of the Compound (4-78) having the following structural formula and the Compound ETM-2 having the following structural formula was formed on the light-emitting layer 5, as the electron transport layer 6 to have a film thickness of 30 nm by binary deposition at a deposition rate in which the ratio of the deposition rates of the Compound (4-78) and the Compound (ETM-1) was 50:50. A film of lithium fluoride was formed, as the electron injection layer 7, on the electron transport layer 6 to have a film thickness of 1 nm. Finally, aluminum was deposited to have a thickness of 100 nm to form the cathode 8. The characteristics of the prepared organic EL device were measured at room temperature in the atmosphere. The measurement results of the light-emitting characteristics when a direct current voltage was applied to the prepared organic EL device were collectively shown in Table 1.

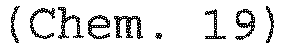

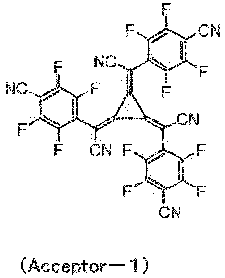

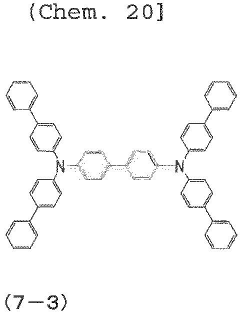

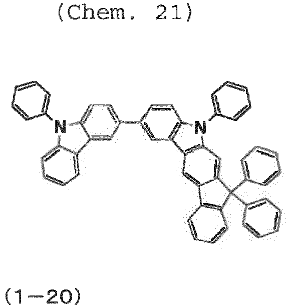

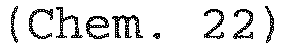

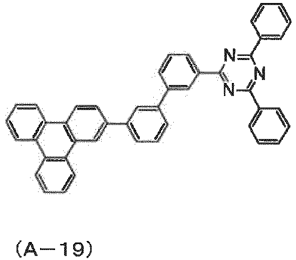

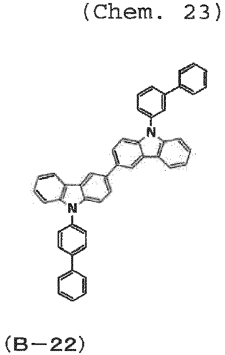

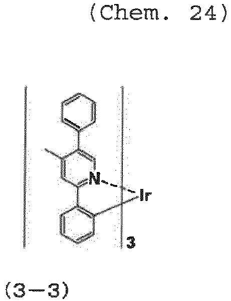

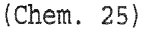

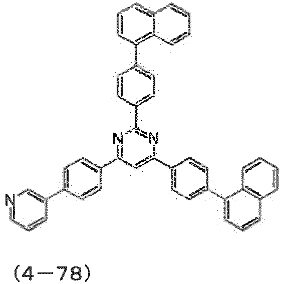

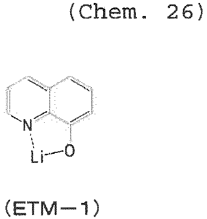

### (Example 11)

An organic EL device was prepared similarly to Example 10 except that the Compound (6-1) was used for the material of an electron transport layer6 instead of the Compound (4-78). The characteristics of the prepared organic EL device were measured at room temperature in the atmosphere. The measurement results of the light-emitting characteristics when a direct current voltage was applied to the prepared organic EL device were collectively shown in Table 1.

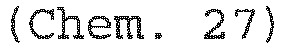

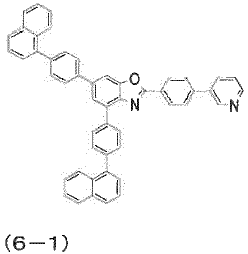

### (Example 12)

An organic EL device was prepared similarly to Example 10 except that the Compound (1-1) according to Example 1 was used for the material of the second hole transport layer 5 instead of the Compound (1-20) according to Example 7. The characteristics of the prepared organic EL device were measured at room temperature in the atmosphere. The measurement results of the light-emitting characteristics when a direct current voltage was applied to the prepared organic EL device were collectively shown in Table 1.

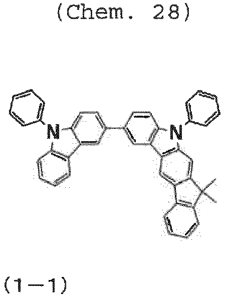

### (Example 13)

An organic EL device was prepared similarly to Example 12 except that the Compound (6-1) was used for the material of the electron transport layer 6 instead of the Compound (4-78). The characteristics of the prepared organic EL device were measured at room temperature in the atmosphere. The measurement results of the light-emitting characteristics when a direct current voltage was applied to the prepared organic EL device were collectively shown in Table 1.

### (Example 14)

An organic EL device was prepared similarly to Example 10 except that the Compound (1-20) according to Example 7 was used as the second host material instead of the Compound (B-22). Here, the first host compound (A-20) and the second host compound (1-20) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

### (Example 15)

An organic EL device was prepared similarly to Example 14 except that the Compound (6-1) was used as the material for the electron transport layer 6 instead of the Compound (4-78). The characteristics of the prepared organic EL device were measured at room temperature in the atmosphere. The measurement results of the light-emitting characteristics when a direct current voltage was applied to the prepared organic EL device were collectively shown in Table 1.

### (Example 16)

An organic EL device was prepared similarly to Example 10 except that the Compound (1-1) according to Example 1 was used as the second host material instead of the Compound (B-22). Here, the first host compound (A-20) and the second host compound (1-1) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

### (Example 17)

An organic EL device was prepared similarly to Example 16 except that the Compound (6-1) was used for the material of the electron transport layer 6 instead of the Compound (4-78). The characteristics of the prepared organic EL device were measured at room temperature in the atmosphere. The measurement results of the light-emitting characteristics when a direct current voltage was applied to the prepared organic EL device were collectively shown in Table 1.

### [Comparative Example 1]

For comparison, an organic EL device was prepared similarly to Example 10 except that the Compound (HTM-2) was used for the material of the second hole transport layer 5 instead of the Compound (1-20) according to Example 7. The characteristics of the prepared organic EL device were measured at room temperature in the atmosphere. The measurement results of the light-emitting characteristics when a direct current voltage was applied to the prepared organic EL device were collectively shown in Table 1.

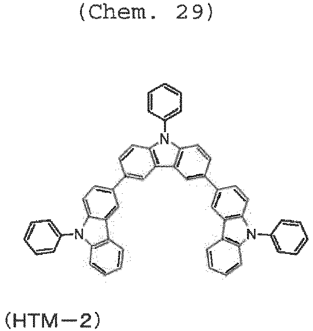

### [Comparative Example 2]

For comparison, an organic EL device was prepared similarly to Example 11 except that the Compound (HTM-2) was used for the material of the second hole transport layer 5 instead of the Compound (1-20) according to Example 7. The characteristics of the prepared organic EL device were measured at room temperature in the atmosphere. The measurement results of the light-emitting characteristics when a direct current voltage was applied to the prepared organic EL device were collectively shown in Table 1.

### [Comparative Example 3]

For comparison, an organic EL device was prepared similarly to Example 10 except that the Compound (B-22) having the following structural formula was used for the material of the second hole transport layer 5 instead of the Compound (1-20) according to Example 7. The characteristics of the prepared organic EL device were measured at room temperature in the atmosphere. The measurement results of the light-emitting characteristics when a direct current voltage was applied to the prepared organic EL device were collectively shown in Table 1.

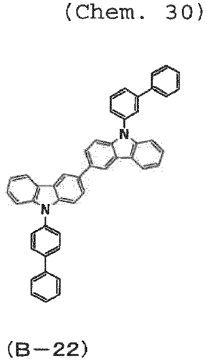

### [Comparative Example 4]

For comparison, an organic EL device was prepared similarly to Example 11 except that the Compound (B-22) was used for the material of the second hole transport layer 5 instead of the Compound (1-20) according to Example 7. The characteristics of the prepared organic EL device were measured at room temperature in the atmosphere. The measurement results of the light-emitting characteristics when a direct current voltage was applied to the prepared organic EL device were collectively shown in Table 1.

The device lifetime was measured using each of the organic EL devices prepared in Examples 10 to 17 and Comparative Examples 1 to 4, and the results were collectively shown in Table 1. The device lifetime was measured as the time until the light emission luminance attenuated to 9500 cd/m² (corresponding to 95% in the case where the initial luminance was 100%: 95% attenuation) when constant current driving was performed with the light emission luminance (initial luminance) at the start of light emission set to 10000 cd/m².

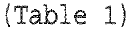

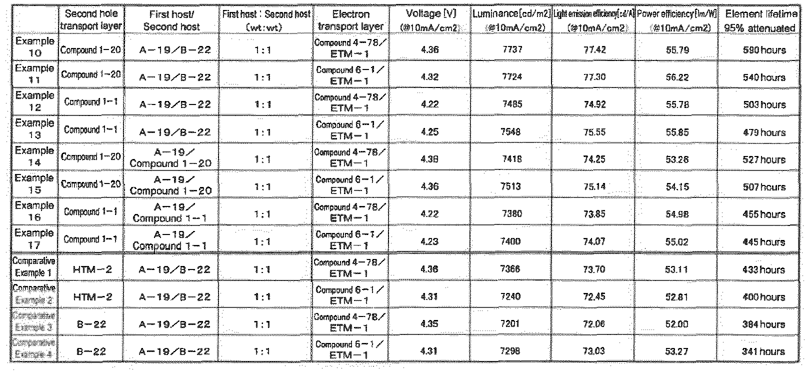

As shown in Table 1, in comparison between Examples 10 to 13 in which the compound having an indenocarbazole ring structure according to the present invention was used as the second hole transport material and Comparative Examples 1 and 2 in which the above-mentioned Compound (HTM-2) was used as the second hole transport material, the light emission efficiency when a current having a current density of 10 mA/cm² was caused to flow was high, i.e., 74.92 to 77.42 cd/A in the organic EL devices according to Examples 10 to 13 as compared with 72.45 to 73.70 cd/A of the organic EL devices according to Comparative Examples 1 and 2. Further, also the power efficiency of the organic EL devices according to Examples 10 to 13 was high, i.e., 55.78 to 56.22 1m/W as compared with 52.81 to 53.11 lm/W of the organic EL devices according to Comparative Examples 1 and 2. Meanwhile, it can be seen that the device lifetime (95% attenuation) was largely extended to 479 to 590 hours in the organic EL devices according to Examples 10 to 13 as compared with 400 to 433 hours of the organic EL devices according to Comparative Examples 1 and 2.

As shown in Table 1, in comparison between Examples 14 to 17 in which the compound having an indenocarbazole ring structure according to the present invention was used as the second hole transport material and the second host material and Comparative Examples 3 and 4 in which the above-mentioned Compound (B-22) was used as the second hole transport material and the second host material, the light emission efficiency when a current having a current density of 10 mA/cm² was caused to flow was high, i.e., 73.85 to 75.14 cd/A in the organic EL devices according to Examples 14 to 17, as compared with 72.06 to 73.03 cd/A of the organic EL devices according to Comparative Examples 3 and 4. Further, also the power efficiency of the organic EL devices according to Examples 14 to 17 was high, i.e., 53.26 to 55.02 lm/W, as compared with 52.00 to 53.27 of the organic EL devices according to Comparative Examples 3 and 4. Meanwhile, it can be seen that the device lifetime (95% attenuation) was largely extended to 445 to 527 hours in the organic EL devices according to Examples 14 to 17 as compared with 341 to 384 hours of the organic EL devices according to Comparative Examples 3 and 4.

As is clear from the results of Table 1, it has been found that an organic EL device that includes a light-emitting layer using both a first host material having high electron transportability and a second host material having hole transportability and uses the compound having an indenocarbazole ring structure according to the present invention as the material of a second hole transport layer is capable of improving power efficiency and prolonging the lifetime even as compared with an organic EL device using the above-mentioned Compound (HTM-2) that is a triscarbazole derivative. By combining the indenocarbazole compound having a specific structure, holes are efficiently supplied to the light-emitting layer, which improves the excess in electrons in the light-emitting layer. As a result, the carrier balance in the light-emitting layer is more refined and an organic EL device with improved efficiency characteristics and remarkably improved lifetime characteristics is realized.

Further, it has been found that an organic EL device using the compound having an indenocarbazole ring structure according to the present invention as a second host material is capable of improving the power efficiency and prolonging the lifetime even as compared with an organic EL device using the above-mentioned Compound (B-22) that is a biscarbazole derivative. By using the indenocarbazole compound having a specific structure as the host material of a light-emitting layer, an organic EL device capable of efficiently injecting/transporting holes to the light-emitting layer is realized. For this reason, an organic EL device with improved efficiency characteristics and remarkably improved lifetime characteristics as compared with the existing organic EL device is realized.

### Industrial Applicability

The organic EL device according to the present invention has improved light emission efficiency and significantly improved durability, and, for example, it has become possible to expand to home appliances and lighting applications.

- 1: glass substrate
- 2: transparent anode
- 3: hole injection layer
- 4: first hole transport layer
- 5: second hole transport layer
- 6: light-emitting layer
- 7: electron transport layer
- 8: electron injection layer
- 9: cathode

## Claims

1. An organic EL device including, between an anode and a cathode, at least a first hole transport layer, a second hole transport layer, a green light-emitting layer, and an electron transport layer in the stated order from a side of the anode, the organic EL device being **characterized in that**
the second hole transport layer, or at least one of stacked films disposed between the first hole transport layer and the electron transport layer contains a compound having an indenocarbazole ring structure, the compound being represented by the following general formula (1). 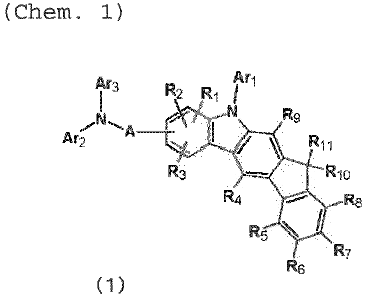 (In the formula, A represents a divalent group of a substituted or unsubstituted aromatic hydrocarbon, a divalent group of a substituted or unsubstituted aromatic heterocycle, or a divalent group of a substituted or unsubstituted fused polycyclic aromatic. Ar₁, Ar₂, and Ar₃ may be the same as or different from each other, and each represent a substituted or unsubstituted aromatic hydrocarbon group, a substituted or unsubstituted aromatic heterocyclic group, or a substituted or unsubstituted fused polycyclic aromatic group. Here, A and Ar₂ or Ar₂ and Ar₃ may form a ring with a single bond or may be bonded to each other via a substituted or unsubstituted methylene group, an oxygen atom, or a sulfur atom to form a ring. R₁ to R₉ may be the same as or different from each other, each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring. R₁₀ and R₁₁ may be the same as or different from each other, each represent a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring.)

2. An organic EL device including, between an anode and a cathode, at least a first hole transport layer, a second hole transport layer, a green light-emitting layer, and an electron transport layer in the stated order from a side of the anode, the organic EL device being **characterized in that** the second hole transport layer, or at least one of stacked films disposed between the first hole transport layer and the electron transport layer contains a compound having an indenocarbazole ring structure, the compound being represented by the following general formula (2). 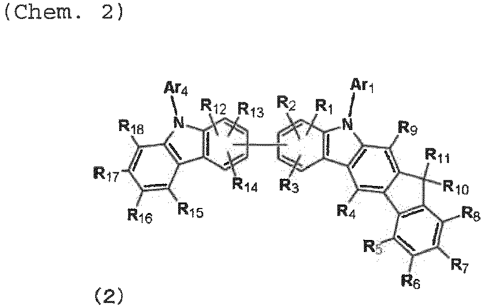 (In the formula, Ar₁ and Ar₄ may be the same as or different from each other, and each represent a substituted or unsubstituted aromatic hydrocarbon group, a substituted or unsubstituted aromatic heterocyclic group, or a substituted or unsubstituted fused polycyclic aromatic group. R₁ to R₉ and R₁₂ to R₁₈ may be the same as or different from each other, each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring. R₁₀ and R₁₁ may be the same as or different from each other, each represent a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and may form a ring with a single bond or may be bonded to each other via a substituted or unsubstituted methylene group, an oxygen atom, or a sulfur atom to form a ring.)

3. The organic EL device according to claim 1 or 2, **characterized in that**
the green light-emitting layer contains a host and a phosphorescent dopant, and the host contains at least one first host compound represented by the following chemical formula Host-A and at least one second host compound represented by the following chemical formula Host-B. 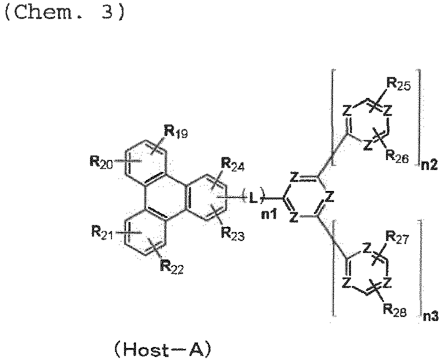 (In the Host-A, Zs each independently represent N or CRa, and at least one of Zs represents N. R₁₉ to R₂₈ and Ra each independently represent a hydrogen atom, a deuterium atom, a fluorine atom, a chlorine atom, a cyano group, a nitro group, a substituted or unsubstituted alkyl group having 1 to 15 carbon atoms, or a substituted or unsubstituted aryl group having 6 to 12 ring carbon atoms. The total number of 6-membered rings substituted with triphenylene groups in the Host-A is six or less. L represents a substituted or unsubstituted phenylene group, a substituted or unsubstituted biphenylene group, or a substituted or unsubstituted terphenylene group. n1 to n3 each independently represent 0 or 1, and n1+n2+n3≥1.) 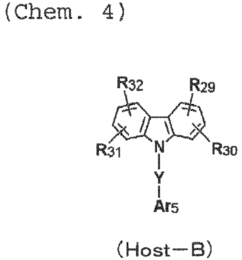 (In the Host-B, Y represents a single bond, a substituted or unsubstituted arylene group having 6 to 30 ring carbon atoms, or a substituted or unsubstituted heteroarylene group having 5 to 30 ring carbon atoms. Ar₅ represents a substituted or unsubstituted aryl group having 6 to 30 ring carbon atoms or a substituted or unsubstituted heteroaryl group having 5 to 30 ring carbon atoms. R₂₉ to R₃₂ each independently represent a hydrogen atom, a deuterium atom, a fluorine atom, a chlorine atom, a cyano group, a nitro group, an alkyl group having 1 to 15 carbon atoms, a substituted or unsubstituted aryl group having 6 to 50 ring carbon atoms, or a substituted or unsubstituted heteroaryl group having 4 to 50 ring carbon atoms. At least one of R₂₉ to R₃₂ and Ar₅ includes a substituted or unsubstituted triphenylene group or a substituted or unsubstituted carbazole group.)

4. The organic EL device according to any one of claims 1 to 3, **characterized in that**
the green light-emitting layer contains a host and a phosphorescent dopant, and the phosphorescent dopant is a metal complex containing iridium.

5. The organic EL device according to any one of claims 1 to 3, **characterized in that**
the green light-emitting layer contains a host and a phosphorescent dopant, and the phosphorescent dopant is a metal complex represented by the following general formula (3). 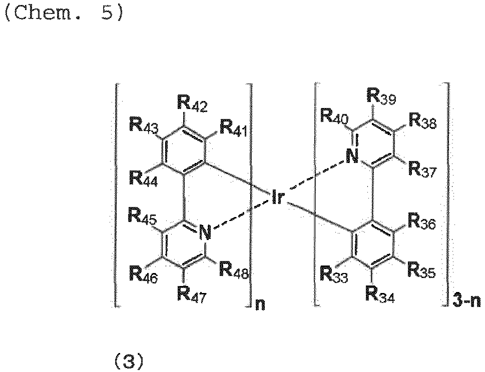 (In the formula, R₃₃ to R₄₈ may be the same as or different from each other, and may each represent a hydrogen atom, a deuterium atom, a fluorine atom, a chlorine atom, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a trimethylsilyl group, a substituted or unsubstituted aromatic hydrocarbon group, a substituted or unsubstituted aromatic heterocyclic group, a substituted or unsubstituted fused polycyclic aromatic group, a substituted or unsubstituted aryloxy group, or a disubstituted amino group substituted with a group selected from an aromatic hydrocarbon group, an aromatic heterocyclic group, or a fused polycyclic aromatic group. n represents an integer of 1 to 3.)

6. The organic EL device according to any one of claims 1 to 5, **characterized in that**
the electron transport layer contains a compound including a pyrimidine structure, the compound being represented by the following general formula (4). 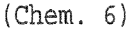 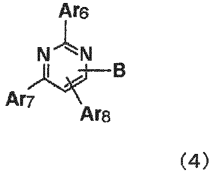 (In the formula, Ar₆ represents a substituted or unsubstituted aromatic hydrocarbon group or a substituted or unsubstituted fused polycyclic aromatic group. Ar₇ and Ar₈ may be the same as or different from each other, and each represent a hydrogen atom, a substituted or unsubstituted aromatic hydrocarbon group, or a substituted or unsubstituted fused polycyclic aromatic group. B represents a monovalent group represented by the following structural formula (5). Here, there is no case that both Ar₇ and Ar₈ are hydrogen atoms.) 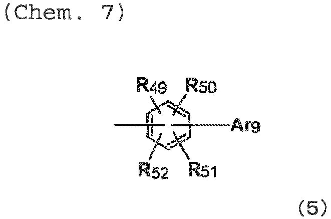 (In the formula, Ar₉ represents a substituted or unsubstituted aromatic heterocyclic group. R₄₉ to R₅₂ may be the same as or different from each other, and each represent a hydrogen atom, a deuterium atom, a fluorine atom, a chlorine atom, a cyano group, a trifluoromethyl group, a linear or branched alkyl group having 1 to 6 carbon atoms, a substituted or unsubstituted aromatic hydrocarbon group, a substituted or unsubstituted aromatic heterocyclic group, or a substituted or unsubstituted fused polycyclic aromatic group.)

7. The organic EL device according to any one of claims 1 to 5, **characterized in that**
the electron transport layer contains a compound having a benzoazole structure, the compound being represented by the following general formula (6). 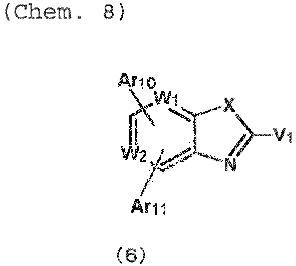 (In the formula, AR₁₀ and AR₁₁ may be the same as or different from each other, and each represent a hydrogen atom, a deuterium atom, a substituted or unsubstituted aromatic hydrocarbon group, a substituted or unsubstituted fused polycyclic aromatic group, or a substituted or unsubstituted aromatic heterocyclic group. V₁ represents a substituted or unsubstituted aromatic hydrocarbon group, a substituted or unsubstituted fused polycyclic aromatic group, a substituted or unsubstituted aromatic heterocyclic group, a linear or branched alkyl group having 1 to 6 carbon atoms which may have a substituted group, a cycloalkyl group having 5 to 10 carbon atoms which may have a substituted group, or a linear or branched alkenyl group having 2 to 6 carbon atoms which may have a substituted group. X represents an oxygen atom or a sulfur atom. W₁ and W₂ may be the same as or different from each other, and each represent a carbon atom or a nitrogen atom.)

8. The organic EL device according to any one of claims 1 to 7, **characterized in that**
the first hole transport layer contains a triphenylamine derivative represented by the following general formula (7) or the following general formula (8) . 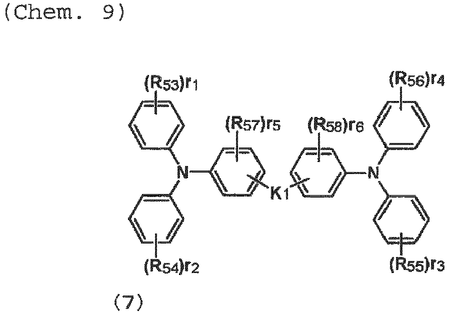 (In the formula, R₅₃ to R₅₈ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₁ to r₆ may be the same as or different from each other, r₁ to r₄ each represent an integer of 0 to 5, and r₅ and r₆ each represent an integer of 0 to 4. In a case where any of r₁ to r₆ is an integer of two or more, a plurality of R₅₃, a plurality of R₅₄, a plurality of R₅₅, a plurality of R₅₆, a plurality of R₅₇, or a plurality of R₅₈ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. K₁ represents a divalent group represented by any of the following structural formulae (HTM-A) to (HTM-F) or a single bond.) 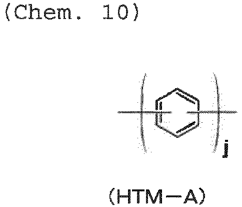 (In the formula, j represents an integer of 1 to 3.) 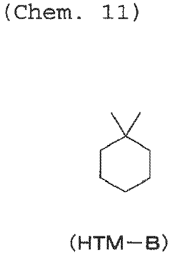 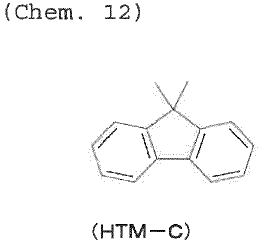 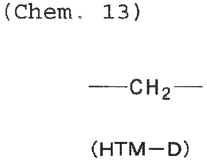 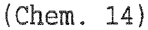 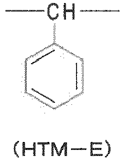 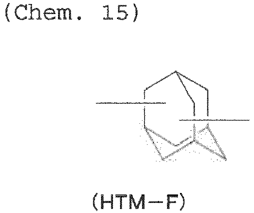 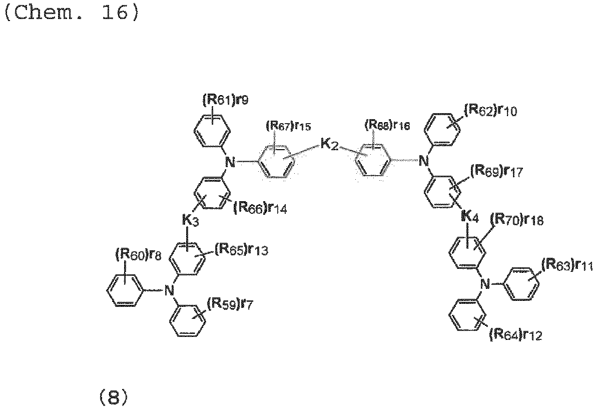 (In the formula, R₅₉ to R₇₀ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₇ to r₁₈ may be the same as or different from each other, r₇ to r₁₂ each represent an integer of 0 to 5, and r₁₃ to r₁₈ each represent an integer of 0 to 4. In a case where any of r₇ to r₁₈ is an integer of two or more, a plurality of R₅₉, a plurality of R₆₀, a plurality of R₆₁, a plurality of R₆₂, a plurality of R₆₃, a plurality of R₆₄, a plurality of R₆₅, a plurality of R₆₆, a plurality of R₆₇, a plurality of R₆₈, a plurality of R₆₉, or a plurality of R₇₀ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. K₂ to K₄ may be the same as or different from each other, and each represent a divalent group represented by any of the structural formulae (HTM-A) to (HTM-F) in the general formula (7), or a single bond.)
